# EUROPÄISCHE PATENTSCHRIFT

(11) **EP 0 797 427 B1**
(45) Veröffentlichungstag und Bekanntmachung des Hinweises auf die Patenterteilung: **14.05.2003**
(21) Anmeldenummer: 95942129.8
(22) Anmeldetag: 12.12.1995
(51) Int. Cl.: A61K 7/48, A61K 7/42, A61K 31/70

(54) **MITTEL GEGEN HYPERREAKTIVE UND HYPOAKTIVE, DEFIZITÄRE HAUTZUSTÄNDE SOWIE MANIFESTE DERMATIDEN**
AGENTS ACTING AGAINST HYPERREACTIVE AND HYPOACTIVE, DEFICIENT SKIN CONDITIONS AND MANIFEST DERMATITIDES
AGENTS EFFICACES CONTRE DES ETATS CUTANES DEFICIENTS HYPER-REACTIFS OU HYPO-ACTIFS ET DES DERMATITES MANIFESTES

(30) Priorität: 13.12.1994 DE 4444238
(43) Veröffentlichungstag der Anmeldung: 01.10.1997
(60) Teilanmeldung: 02025465.2
(73) Patentinhaber: Beiersdorf Aktiengesellschaft, 20245 Hamburg (DE)
(72) Erfinder: LANZENDÖRFER, Ghita, D-22087 Hamburg (DE); STÄB, Franz, D-21379 Echem (DE); UNTIEDT, Sven, D-20259 Hamburg (DE)
(86) Internationale Anmeldenummer: EP9504907
(87) Internationale Veröffentlichungsnummer: WO96018381

(56) Entgegenhaltungen:
- EP-A- 0 387 042
- EP-A- 0 402 049
- EP-A- 0 496 649
- EP-A- 0 577 143
- EP-A- 0 595 694
- FR-A- 2 699 818
- GB-A- 2 259 014
- PATENT ABSTRACTS OF JAPAN vol. 13 no. 308 (C-617) [3656] ,14.Juli 1989 & JP,A,01 096106 (SHISEIDO CO LTD) 14.April 1989,
- PATENT ABSTRACTS OF JAPAN vol. 12 no. 496 (C-555) [3343] ,23.Dezember 1988 & JP,A,63 208506 (NONOGAWA SHOJI K.K.) 30.August 1988,

## Beschreibung

Die vorliegende Erfindung betrifft insbesondere Wirkstoffe und Zubereitungen, insbesondere zur topischen Anwendung, die zur Prophylaxe und Behandlung bei hyperreaktiver und für Dermatiden prädisponierter Haut sowie defizitärer, hypoaktiver Haut und zur Prophylaxe und Behandlung der unter I. bis XIII. benannten manifesten Dermatosen wie z.B. Atopische Dermatitis, Neurodermitis, Atopisches Ekzem und seborrhoische Dermatitis, lichtinduzierter Dermatosen ( z. B. Mallorca Akne und insbesondere Polymorphe Lichtdermatose, Photodermatitis), Rosacea, Prurigo-Formen, Pruritus, Psoriasis-Formen, Ichthyose, Decubitus, Ulcus cruris sowie mikrobielle und viralen Infektionen wie z. B. Herpes simplex, H. Zoster oder H. labialis dienen.

Die erfindungsgemäßen Hautzustände werden im folgenden näher erläurtert.

Verschiedene pathologische Mechanismen, die das klinische Bild bei hypoaktiver Haut oder hyperreaktiver Haut und bei den unter I. bis XIII. genannten Dermatosen, wie zum Beispiel bei Akneformen, bei Atop.

Dermatiden, Prurigo-Formen, Psoriasis, Photodermatosen, Decubitus, Ulcus cruris und Ichthyose prägen, werden in der Literatur beschrieben. Jedoch sind bei keiner der genannten Dermatosen die causalen pathologischen Mechanismen und deren Chronologie hinreichend bekannt. Die bisher bekannten Behandlungsmethoden und verabreichten Wirkstoffe führen günstigstenfalls zu einer kurzzeitigen Verbesserung der Symptome. Zur Behandlung werden z.B. UV-Therapie und /oder Chemotherapie (Psoralen, PUVA, Cyclosporin A, Kortikosteroide, Acyclovir etc.) angewandt mit den bekannten negativen Nebenwirkungen bei wiederholter Applikation.

Aus der EP-A-0387 042 ist bereits die Behandlung von Brandwunden mit Alphaglycosylrutin bekannt.

Aufgabe der Erfindung ist es, diesen unbefriedigenden Stand der Technik zu verbessern und kosmetische, dermatologische und/oder pharmazeutische Wirkstoffe und Zubereitungen zu schaffen, die zur Prophylaxe und Behandlung bei defizitärer, hypoaktiver Haut oder zur Prophylaxe und Behandlung bei hyperreaktiver, zu Dermatiden neigender Haut und zur Prophylaxe und Behandlung bei den im folgenden unter den Abschnitten I. bis XIII. genannten manifesten Dermatosen dienen, ohne die Nebenwirkungen bekannter Mittel, auch bei dauerhafter Anwendung, zu induzieren.

Diese Aufgaben werden gemäß der Erfindung gelöst.

Gegenstand der Erfindung ist die Verwendung von
a) einer Verbindung oder mehrerer Verbindungen aus der Gruppe der Flavonoide oder
b) einer Wirkstoffkombination, enthaltend eine Verbindung oder mehrere Verbindungen, ausgewählt aus der Gruppe der Flavonoide in Kombination mit einer Verbindung oder mehreren Verbindungen, ausgewählt aus der Gruppe der Zimtsäurederivate, und gegebenenfalls zusätzlich jeweils einer Verbindung oder mehreren Verbindungen aus einer der Gruppen oder mehreren der Gruppen
c) der Antioxidantien oder
d) der endogenen Energiestoffwechselmetaboliten oder
e) der endogenen enzymatischen Antioxydansysteme und deren synthetischen Derivaten (Mimics) oder
f) der antimikrobiellen Wirksysteme oder
g) der antiviralen Wirksysteme oder
h) Wirkstoffen der bekannten, konventionellen Behandlungsformen
jeweils zur Herstellung eines dermatologischen und/oder pharmazeutischen Mittels zur Behandlung oder prophylaktischen Behandlung von hyperreaktiver und für Dermatiden prädisponierter Haut oder defizitärer, hypoaktiver Haut oder Dermatosen.

Wirkstoffkombinationen b), deren Verwendung und Zubereitungen, die diese enthalten, werden bevorzugt.

Gegenstand der Erfindung sind auch kosmetische und dermatologische Zubereitungen, insbesondere topische Zubereitungen und pharmazeutische Präparate mit einem Gehalt an den vorstehend genannten Wirkstoffen zur Behandlung und prophylaktischen Behandlung der vorstehend genannten Hautzustände oder Krankheiten.

Ebenfalls sind Gegenstand der Erfindung kosmetische und dermatologische, insbesondere topische Zubereitungen und pharmazeutische Präparate mit einem Gehalt an den vorstehend angegebenen erfindungsgemäßen Wirkstoffen.

Die erfindungsgemäßen manifesten Dermatosen bzw. Hautkrankheiten (Dermatiden) und deren wichtigste Formen und Benennungen sind insbesondere:
**I. Atopisches Ekzem:**
   Neurodermitis
   atopische Dermatitis, dermatitis atopica
   atopisches Ekzem mit Typ I und Typ IV Kontaktekzem, berufsbedingt aggravierend
   Windeldermatitis
   Milchschorf
   Ekzematöse Erythrodermie
**II. Kontaktekzem:**
   toxisches/irritantes Kontaktekzem, berufsbedingt (z.B. Öl/Teer; Halogene)
   allergisches Kontaktekzem, Typ I oder Typ IV
   Photoallergisches Kontaktekzem
   Kontakturtikaria
   Dyshidrosiformes Ekzem
**III. Akne:**
   Akne vugaris, juvenil und adult, (komedonenakne, papulöse, pustulöse, knotige d. h. noduläre, nodulozystische Akne)
   Akne conglobata, (Sonderform: Hidradenitissupprativa)
   Akne fulminans
   Akne - Tetrade
   Akne neonatorum
   Altersakne (M. Favre - Racouchot)
   Mechanische Akneformen(Acne excoriee)
   Akne cosmetica
   Follikulitis bei superinfizierter Akne (Stapylokokken) berufsbedingte Akneformen (z. B. Chlorakne)
**IV. Herpesvirus - Infektionen:**
   Herpes simplex (HSV I+ II)
   Herpes zoster (varizellavirus
   Herpes labialis (Gingivostomatitis herpetica, Mundfäule, Aphten, Stomatitis aphtosa,
   Paronchyia herpetica, Ekzema herpeticatum, Aphtoid von Pospiscill und Feyrter, Vulvovaginitis
   herpetica,Keratoconjunctivitis herpetica, herpes simplex recidivans, herpes genitalis recidivans, Herpes glutaelis recidivans, Dermatitis herpetiforme)
   Varizellavirus (Herpes zoster, Gürtelrose, Shingles, Zona)
**V. Bakterielle Infektionen:**
   Nichtfollikuläre Pyodermien, vulgaris oder bullös (Impedigo contagiosa Streptokokken, Staphylokokken)
   Follikuläre Pyodermien (Kokken), Furunkel, Karbunkel, Follikulitis z. B. F. barbae
   Mykobakteriosen (Korynebakterien, Spirochäten, Anaerobier)
   Erysipel (grampos. Kokken, gramneg. Stäbchen)
   Ecthyma - Formen
   Lepra- Formen
   Erythrasma (Coryneb. minutissimum)
   Trichomykosis axillaris (Coryneb. tenuis)
**VI. Psoriasis:**
   Psoriasis vulgaris
   Schuppenflechte
   Psoriasis pustulosa
   Psoriasis arthropatica
   psoriatische Erythrodermie
**VII. Rosacea**
**VIII. Periorale Dermatitis**
**IX. Prurigo:**
   P. simplex acuta (Strophulus, Urticaria papulosa), subacuta, chronica,
   P. nodularis Hyde
**X. Ekzem:**
   Seborrhoisches Ekzema (P. ovale)
   Mikrobielles Ekzem (Windeldermatitis- Bacillus amoniagenese, B. proteus, B. subtilis, E. coli, S. aureus)
   Numuläres Ekzem
   Dyshidrotisches Ekzem
   Exsikkationsekzem (asteatotischesEkzem, Xerosis, Eczema craquele)
   Lichen simplex chronicus (Neurodermitis circumscripta)
**XI. Lichtdermatose:**
   Radiodermatitis acuta und chronica ( UV- und ionisierende Strahlentherapie)
   Chronisch - aktinische Dermatitis
   Lichturticaria (Urticaria solaris)
   Polymorphe Lichtdermatose ( Weitere Bezeichnungen für die polymorphe Lichtdermatose sind PLD, PLE und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.)
**XII. Decubitus**
**XIII. Ulcus cruris**

Im folgenden werden die erfindungsgemäßen Flavonoide auch mit A), die Zimtsäurederivate auch mit B), die Antioxidantien auch mit C), die endogenen Energiestoffwechselmetaboliten auch mit D), die endogenen enzymatischen Antioxydanssysteme oder Stoffe und deren synthetische Derivate (Mimics) auch mit E), die antimikrobiellen Wirksysteme oder Wirkstoffe auch mit F) die antiviralen Wirksysteme oder Stoffe auch mit G) und die bekannten konventionellen Behandlungsformen oder Stoffe auch mit H) bezeichnet.

Bevorzugte erfindungsgemäße Flavonoide sind beispielsweise hydroxylierte Flavone, Flavanone, Isoflavone oder Chalcone und jeweils auch deren Glycoside, aber auch diese nicht hydroxylierten Grundstrukturen bzw. Stammsubstanzen.

Erfindungsgemäß werden die Flavonoide A) bevorzugt gewählt aus der Gruppe der Substanzen der generischen Strukturformeln: 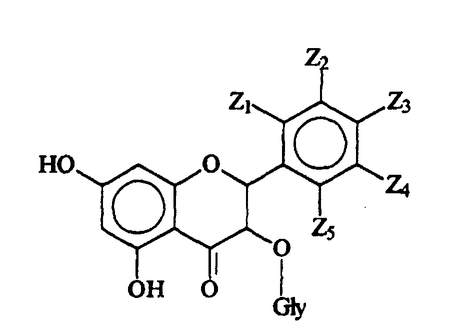 und 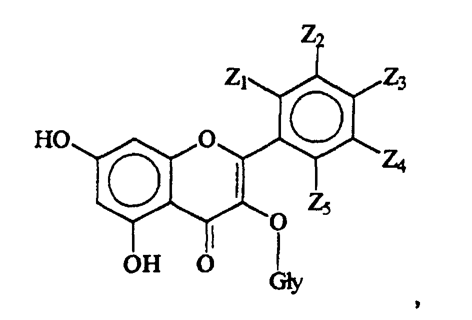 wobei Z₁ - Z₅ unabhängig voneinander gewählt werden aus der Gruppe H, OH und O-Alkyl, wobei die Alkylgruppen verzweigt und unverzweigt sein und 1 - 18 C-Atome aufweisen können, und wobei Gly gewählt wird aus der Gruppe der Mono- und Oligoglycosidreste oder auch H darstellen kann. Bevorzugte Glycosidreste sind die für Gly₁ - Gly₃ nachstehend angegebenen.

Weitere erfindungsgemäße Flavonoide werden bevorzugt gewählt aus der Gruppe der Substanzen der folgenden Formeln: 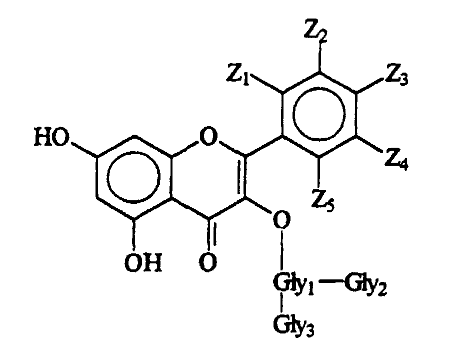 sowie 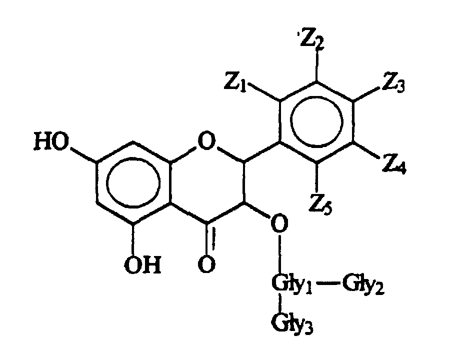 worin Z1 bis Z5 die oben angegebene Bedeutung haben und Gly₁, Gly₂ und Gly₃ Monoglycosidreste darstellen.

Bevorzugt werden Gly₁, Gly₂ und Gly₃ unabhängig voneinander gewählt aus der Gruppe der Hexosylreste, insbesondere der Rhamnosylreste und Glucosylreste. Aber auch andere Hexosylreste, beispielsweise Allosyl, Altrosyl, Apiosyl, Arabinosyl, Biosidyl, Galactosyl, Gulosyl, Glucoronidyl, Idosyl, Mannosyl, Talosyl und Xylosyl sind gegebenenfalls vorteilhaft zu verwenden. Es kann auch erfindungsgemäß vorteilhaft sein, Pentosylreste zu verwenden.

Es kann ebenfalls vorteilhaft sein auf die oben erwähnten glycosidischen Reste Gly₁₋₃ zu verzichten und die nicht substituierten Flavonoide (Gly₁₋ ₃ = H), wie z. B. Quercitin, zu verwenden. Ebenso kann es von Vorteil sein Flavonoide zu verwenden, deren Glucosid-Rest über phenolische OH-Funktionen an C7, C4', C3' oder C5' gebunden ist.

Weiterhin kann es von Vorteil sein, Flavonoide zu verwenden, deren phenolische OH-Funktion an C9 frei vorliegt (sogenannte Chalkone). Insbesondere ist es vorteilhaft aus dieser Gruppe Neohesperidin Dihydrochalkon zu verwenden.

Erfindungsgemäß sind weiterhin Verbindungen von Flavonoiden oder Flavonglycosiden mit Derivaten der o. g. Zuckerreste wie 1->2; 1->3; 1->4 oder 1->5 Di-, Oligo- oder Poly -alpha- oder -betaglycosidische Verbindungen gleicher oder verschiedener Zucker untereinander sowie Zuckersäuren, sowie Zuckerestern, sowie substituierten Zuckern z. B. mit NR1R2, wobei R1 = H, alkyl und R2 = H, alkyl sein kann (z. B. N-Acetylglucosamin).

Vorteilhaft im Sinne der vorliegenden Erfindung ist es, das oder die Flavonglycoside zu wählen aus der Gruppe Quercitin, Rutin, Chrysin, Kaempferol, Myricetin, Rhamnetin, Apigenin, Luteolin, Naringin, Hesperidin, Naringenin, Hesperitin, Morin, Phloridzin, Diosmin, Fisetin, Vitexin, Neohesperidin Dihydrochalkon, Flavon, Glucosylrutin und Genistein.

Die erfindungsgemäß besonders bevorzugten Flavonglucoside sind Chrysin, Naringin, Hesperidin, Naringenin, Hesperetin, Morin, Phloridzin, Diosmin, Neohesperidin Dihydrochalkon, Flavon und alpha-Glucosylrutin der Formel

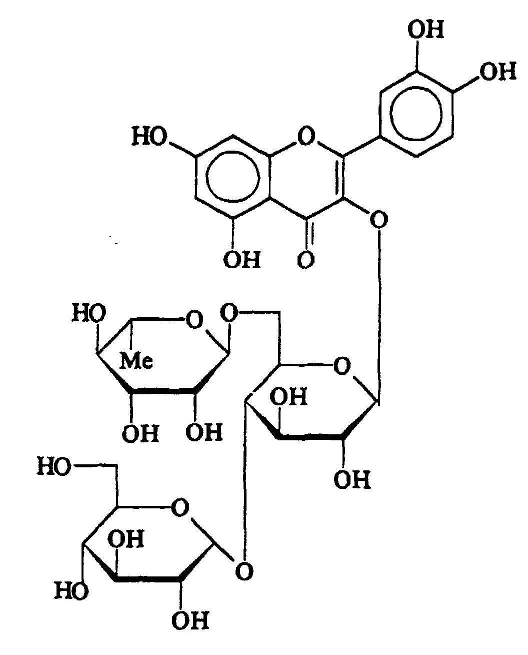

Das erfindungsgemäß bevorzugte Flavonglucosid ist alpha-Glucosylrutin. Es zeichnet sich insbesondere durch die angegebene Struktur aus.

Besonders vorteilhaft im Sinne der vorliegenden Erfindung ist, das oder die Flavonglycoside zu wählen aus der Gruppe alpha-Glucosylrutin, alpha-Glucosylmyrictrin, alpha-Glucosylisoquercitrin und alpha-Glucosylquercitrin.

Darüberhinaus sind besonders erfindungsgemäß bevorzugt Verbindungen wie alpha - Glycosylrutin, alpha-Glycosylhesperidin, alpha-Glycosylnaringin, alpha-Mannosylrutin, alpha-Rhamnosyirutin.

Ferner kann es vorteilhaft sein im Sinne der Erfindung handelsübliche, flavonoidhaltige Pflanzenextrakte zu verwenden. Bei diesen kann es sich um nach den üblichen Methoden gewonnene wassrig-alkoholische bzw. wässrig-glykolische Extrakte sowie trockene Extrakte handeln.

Insbesondere als vorteilhaft haben sich erwiesen: Zitrusfruchtschalen- oder -kernextrakt (z. B. Citricidal/Fa. Synthapharm), Soyaextrakt (z. B. Phytodermin/Fa. Chem. Laboratorium Dr. Kurt Richter GmbH), Sophora Japonica-Extrakt (z. B. Sophorine/Fa. Solabia), Frauendistelextrakt (z. B. Psoralen Silymarin/Fa. Mani GmbH Chemische Produkte), Katzenpfötchenblütenextrakt, Spinatextrakt und ein gemischter Pflanzenextrakt aus Passionsblumen, schwarzen Johannisbeeren und Weinbättern (AE Complex/Fa. Solabia), Calendula-Extrakt(Pot Marigold AMI watersoluble/Alban Muller).

Geeignete Zimtsäurederivate sind z.B. Hydroxyzimtsäuren und deren Derivate, wobei die Derivate z.B. die im folgenden definierten sein können.

Erfindungsgemäß können Zimtsäurederivate der allgemeinen Formel 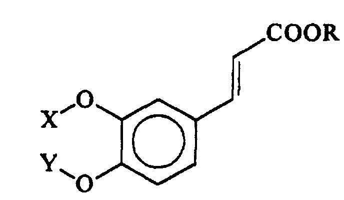 und/oder wirksame Mengen an Zimtsäurederivaten der allgemeinen Formel 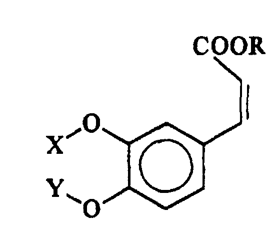 verwendet werden, wobei die Gruppen X, Y und R unabhängig voneinander gewählt werden können aus der Gruppe H, verzweigtes bzw. unverzweigtes Alkyl mit 1 - 18 C-Atomen.

Es können die Säuren oder deren Salze verwendet werden, vorzugsweise die physiologisch verträglichen Salze, beispielsweise wassserlösliche Salze (Natrium-, Kaliumsalze).

Als besonders vorteilhaftes Zimtsäurederivat im Sinne der vorliegenden Erfindung wird die Ferulasäure angesehen. Ferulasäure (4-Hydroxy-3-methoxyzimtsäure, Kaffeesäure-3-methylether) ist durch die Strukturformel 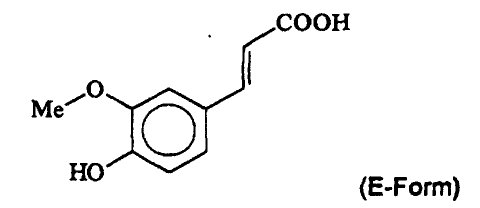 bzw. 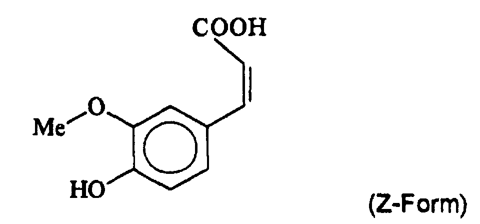 gekennzeichnet. Sie ist in Pflanzen weit verbreitet und kommt z.B. in Rüben, Getreide und dem Milchsaft der namensgebenden Doldenblütlern *Ferula asafoetida* und *Ferula narte*x vor. Die E-Form ist unter Normalbedingungen ein farblos-kristalliner Festkörper, die Z-Form liegt unter Normalbedingungen als gelbliches Öl vor.

Im Sinne der vorliegenden Erfindung ist bevorzugt E-Ferulasäure zu verwenden. Es ist jedoch gegebenenfalls auch von Vorteil, Z-Ferulasäure bzw. beliebige Gemische aus E- und Z-Ferulasäure einzusetzen.

Eine weiteres erfindungsgemäß bevorzugtes Derivat der Zimtsäure ist die Kaffeesäure, welche sich durch die Struktur 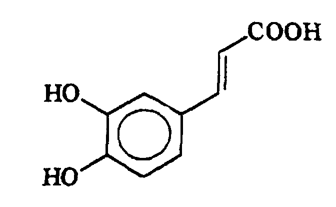 auszeichnet. Sie ist eine weit verbreitete Pflanzensäure und z.B. in Kaffee, Tabak, Mohn und Löwenzahn enthalten.

Es ist auch gegebenenfalls vorteilhaft Pflanzenextrakte mit einem Gehalt an erfindungsgemäßen Zimtsäurederivaten, insbesondere Ferulasäure und/oder Kaffeesäure, zu verwenden.

Unter dem Begriff "Derivate der Kaffeesäure oder Ferulsäure" sind zu verstehen ihre kosmetisch oder pharmakologisch unbedenklichen Ester, Salze und Basenaddukte, insbesondere solche, wie sie bei den Zimtsäurederivaten vorstehend beschrieben sind.

Bevorzugt erfindungsgemäße Kombinationen sind Kombinationen von einem oder mehreren Stoffen aus der Gruppe der oben aufgeführten Flavonoide oder Kombinationen von einem oder mehreren Vertetern der Flavonoide mit einem Derivat der Zimtsäure oder auch die Kombination mit mehreren Zimtsäurederivaten.

Erfindungsgemäß besonders bevorzugt sind die Kombinationen von Flavonoiden, Flavonglucosiden bzw. flavonoidhaltigen pflanzlichen Extrakten mit Ferulasäure sowie die Kombination von synthetisch modifizierten, insbesondere glykosylierten Flavonoiden wie alpha-Glukosylrutin mit Zimtsäurederivaten.

Das Gewichtsverhältnis der Zimtsäurederivate zu dem oder den Flavonoiden beträgt vorteilhaft 25 : 1 bis 1 : 25, bevorzugt 5 : 1 bis 1 : 5, insbesondere bevorzugt etwa 2 : 1 bis 1 : 2.

Die erfindungsgemäßen kosmetischen und dermatologischen Zubereitungen enthalten bevorzugt 0,001 Gew.-% bis 30 Gew.-%, vorzugsweise 0,01 Gew.-% bis 10 Gew.-%, insbesondere aber 0,1 Gew.-% bis 6 Gew.-%, bezogen auf das Gesamtgewicht der Zubereitungen eines oder mehrerer erfindungsgemäßer Stoffe A) oder der Kombination von A) und B).

Besonders bevorzugt werden Zubereitungen mit Kombinationen b), die alpha-Glucosylrutin und/oder Ferulasäure enthalten.

In den erfindungsgemäßen Zubereitungen können als alleinige Wirkstoffe die Verbindungen der Gruppe A oder die der Kombination der Wirkstoffe A) und B) vorliegen.

Die erfindungsgemäßen Zubereitungen können aber bevorzugt neben den Wirkstoffen A oder der Kombination von A) und B) auch noch einen Gehalt an einem Antioxidans oder mehreren Antioxidantien C) haben.

Vorteilhaft können die erfindungsgemäßen Antioxidantien C) aus der Gruppe der Tocopherole und deren Derivaten ausgewählt werden. Die Tocopherole, auch Vitamin E genannt, leiten sich vom Grundkörper Tocol (2-Methyl-2-(4,8,12-trimethyltridecyl)chroman-6-ol) ab. Dem natürlich am häufigsten vorkommenden und bedeutendsten alpha-Tocopherol kommt die Konfiguration 2R,4'R,8'R zu. Es wird gelegentlich auch RRR-alpha-Tocopherol genannt.

Die erfindungsgemäß bevorzugten Tocopherolderivate sind das alpha-Tocopherol und seine Ester, insbesondere das alpha-Tocopherylacetat. Ester von Säuren mit 2 - 18, insbesondere 2 - 8 C-Atomen werden bevorzugt.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Weitere bevorzugte erfindungsgemäße Antioxidantien C) und antioxidative Wirkstoffe sind:
L-Aminosäuren (z.B. von Glycin, Histidin, Tyrosin, Tryptophan, Phenylalanin, Methionin, Glutaminsäure, Arginin, Serin) und deren Derivate (z. B. Hydroxyl-, Methyl-, Ethyl-Verbindungen), Imidazole (z.B. Urocaninsäure) und deren Derivate, Peptide mit einem Anteil an L-Histidin wie z. B. D,L-Carnosin, D-Carnosin, L-Carnosin und deren Derivate (z.B. Anserin, L-Alanylhistidin, L-Histidyltryptophan, L-Methionylhistidin, L- Histidyltyrosyltryptophan, Methionyltryptophan) und/oder einem Anteil an Tryptophan (z. B. L-Glycyltryptophan, L-Tryptophanylhistidin, L-Methionyl-tryptophan), Polyamine (z. B. Spermin, Spermidin), Carotinoide, Carotine (z.B. alpha-Carotin, β-Carotin, Lycopin) und deren Derivate, Liponsäure und deren Derivate (z.B. Dihydroliponsäure), Aurothio-glucose, Propylthiouracil, Oxothiazolidin-4-carboxylat, Thioharnstoff und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl - und Glycerylester) sowie deren Salze, Dilaurylthiodipropionat, Distearylthiodipropionat, Thiodipropionsäure und deren Derivate (oxidierte und/oder reduzierte Formen, Ester, Ether, Peptide, Lipide, Nukleotide, Nukleoside und Salze) sowie Sutfoximinverbindungen (z.B. Buthioninsulfoximine, Homocysteinsulfoximin, Buthioninsulfone, Pentat-, Hexa-, Heptathioninsutfoximin) in sehr geringen verträglichen Dosierungen (z.B. pmol bis mmol/kg). Ferner (Metall)-Chelatoren (z.B. alpha-Hydroxyfettsäuren, Palmitinsäure, Phytinsäure, Lactoferrin), alpha-Hydroxysäuren, (Z.B.Zitronensäure, Milchsäure, Äpfelsäure, Salicylsäure, Glyoxylsäure), Huminsäure, Gerbsäuren,Tannine, Gallensäure und deren Derivate (z. B. Cholsäure, Glucocholsäure, Taurocholsäure, Taurin), Gallenextrakte, Bilirubin, Biliverdin, EDTA, EGTA und deren Derivate sowie ungesättigte Fettsäuren und deren Derivate (z.B. gamma-Linolensäure, Linolsäure, Ölsäure), Folsäure und deren Derivate, Ubiquinon und Ubiquinol deren Derivate, Vitamin C und Derivate (z.B. Ascorbylpalmitate, Mg - Ascorbylphosphate, Ascorbylacetate, Ascorbylglycoside), Tocotrienole, Tocopherole und deren Derivate (z.B. Vitamin E - acetat, alpha-, beta-, gamma-, delta-Tocopherole, Tocopherylglycoside), Vitamin A und Derivate (Retinol, Vitamin A - palmitat, Vitamin A - Säure) sowie Konyferylbenzoat des Benzoeharzes, wässrige oder alkoholische Tabak-, Tee- und/oder Kaffee - Extrakte, Teein, Coffein, Chlorogensäure, Nikotin, Nikotinsäure, Quercitin, Myricitin, Gingko biloba - Extrakte, Cucumberaceen-Extrakte (z. B. von Gurken), Brassicaceen-Extrakte (z. B. von Kohl-pflanzen), Kamillenextrakt, Thymianextrakt, Rosmarinextrakt, Kampferol,Benzoesäure und deren Derivate(z. B. Ethyl-, Isopropyl-, Propylgallat), Butylhydroxytoluol, Butylhydroxyanisol, Nordihydroguajrekharzsäure, Nordihydroguajaretsäure, Trihydroxybutyrophenon, Harnsäure, Harnstoff und deren Derivate, Mannose, Glucose, Galactose, Fructose und deren Derivate (z. B. -6-phosphat, 1-6-diphosphat, Dextrane, Glucane, insbesondere beta-Glucane), Zink und dessen Derivate (z.B. ZnO, ZnSO₄), Selen und dessen Derivate (z.B. Selenmethionin), Stilbene und deren Derivate (z.B. Stilbenoxid, Trans-Mgcl₂) und die erfindungsgemäß geeigneten Derivate (Salze, Ester, Ether, Alkohole, Zucker, Nukleotide, Nukleoside, Peptide und Lipide) Stilbenoxid), Calcium und Magnesium und deren Derivate (z B. Cacl₂, dieser genannten Wirkstoffe.

Die Menge der Wirkstoffkomponenten C) (eine oder mehrere Verbindungen) in den Zubereitungen beträgt vorzugsweise 0,001 Gew.% bis 30 Gew.%, besonders bevorzugt 0,05 - 20 Gew.%, insbesondere 1 - 10 Gew.-%, bezogen auf das Gesamtgewicht der Zubereitung. Diese antioxidativen Wirkstoffe C) können erfindungsgemäß mit einem oder mehreren Wirkstoffen von A) und B) sowie von D) bis H) in den dort angegebenen Konzentrationen kombiniert werden.

Erfindungsgemäß bevorzugte, den Energiestoffwechsel und die endogenen, enzymatischen Antioxidanssysteme, insbesondere in der Haut, günstig beeinflussende und regulierende Wirkstoffe D) sind z.B. Vitamin D und dessen Derivate (z. B. Vitamin D₃), Melatonin und dessen Derivate, D-Biotin und dessen Derivate (z. B. Biotin-ethyl, -methyl, butyl-, -propionyl, -isopropionyl-ester, Biocytin), Glucose und Glucose-Derivate (z.B. Glucose-6-phosphat, Glucose-1,6-phosphat, Glucosylcystein, Glucosylcystin, Glycosylcysteine, Glycosylcystine, Glucosylgluthation, Glucosylcystamin, Glycosylcystamin), Pyruvat, Coenzym A und dessen Derivate, Coenzym Q, Ubiquinol und deren Derivate, Niacinsäure, NADH, NADPH, Adenin, Adenosin, Methyl-S-adenosin, cAMP, ADP, ATP, Guanin, Guanosin, cGMP, GDP, GTP und FAD⁺, FADH₂, FMN, Folsäure, Dihydrofolat, Riboflavine, Panthotensäure, Panthenol, Thiaminpyrophosphat, Thiamin, 6 - Phos-phoglucurono-delta-lacton, 6 - Phosphogluconsäure, Fructose-6-phosphat, Glycerin-aldehyd - 3-phosphat, Ribulose - 5 - phosphat, Pyridoxamin, Pyridoxalphosphat, Biopterine (z. B. Aminopterin, Tetrahy-dropterin), alpha- Hydroxysäuren (z. B. Milchsäure, und die geeignete Derivate (Salze, Zucker, Ester, Ether, Nukleotide, Nukleoside, Peptide und Lipide) der genannten Wirkstoffe.

Die Menge dieser den Energiestoffwechsel regulierenden Wirkstoffe D) beträgt z.B. 0,0001 Gew.-% bis 30 Gew.-%, vorzugsweise 0,01 Gew.-% bis 20 Gew.-%, insbesondere 0,1 Gew.-% bis 10 Gew.-%, jeweils bezogen auf das Gesamtgewicht der Zubereitungen. Diese den Energiestoffwechsel reulierenden Wirkstoffe D) können erfindungsgemäß mit Wirkstoffen von A) bis C) sowie E) bis H) erfindungsgemäß in den dort jeweils angegebenen Konzentrationen kombiniert werden.

Erfindungsgemäß wirksame enzymatische Antioxidanssysteme E) (z.B. in Zubereitungen in Mengen von 0,0001 - 15 Gew. %) und deren synthetische Mimics sind z. B. die Enzyme Superoxiddismutase, Glutathionperoxidase, Glutathionreductase, Glutathion-S-Transferase, Oxidoreductasen (z. B. Glucose-6-phosphat - dehydrogenase, Succinatdehydrogenase, Malatdehydrogenase, insbesondere die NAD⁺-, NADP⁺- und FAD⁺- Reduktasen sowie Carboxylasen (z. B. Biotin-carboxylase, Acetyl-CoA Carboxylase), Cytochrom P 450- Reductase, Cytochrom P450 - Monooxigenasen, Monoaminoxidase und insbesondere die erfindungsgemäß wirksamen Coenzymen (0,0001 - 5 Gew. %) und deren Vorstufen (z. B. Provitamine, Vitamine) zu den genannten Enzyme.

Bevorzugt beträgt die Menge der erfindungsgemäß wirksamen enzymatischen Antioxydantien E) in den Zubereitungen 0,001 Gew.% bis 40 Gew.-%, vorzugsweise von 0,01 Gew.-% bis 30 Gew.%, insbesondere 0,1 Gew.% bis 15 Gew.% bezogen auf das Gesamtgewicht der Zubereitung. Die erfindungsgemäß wirksamen enzymatischen Antioxidantssysteme E) können erfindungsgemäß mit einem oder mehreren Wirkstoffen von A) bis D) und F) bis H) in den dort angegebenen Konzentrationen kombiniert werden.

Erfindungsgemäß können (insbesondere auch in Kombination mit einem oder mehreren Wirkstoffen aus A) bis E) und G) bis H)) antibakterizide und antibakteristatische Wirkstoffe F) verwendet werden, wie z. B. Vanilinsäure,Gentianaviolett, Amonium - Tumenol, Tyrotricin/Cetylpyridiniumchlorid, Dequaliniumchlorid, Aluminiumchlorid, Chlorhexidingluconat, Chinolinol, Thymol, Policresulen, Wollwachssäuren und deren Derivate, Lanthabiotika (wie z. B. Nisin), Triclosan, Minocyclin, Doxycyclin,Tetracyclin, omega und/oder alpha - Fettsäuren (z. B.C₅ bis C₁₆ -kettige Fettsäuren sowie gamma-Linolensäure, Linolsäure) und deren Ester und Alkohole (z. B. Monoglycerin-, Diglycerin-, Triglycerinester, Ethyl-, Methyl-, Propyl-, Isopropyl-, Butylester), Hamamelis - Extrakt, Salicylsäure, Azelainsäure und deren Derivate, Sulfonamide sowie Antimykotika wie z. B. Imidazolderivate (z. B. Clotrimazol, Econazol, Oxiconazol, Miconazol, Ketoconazol, Isoconazol), Griseofulvin, Terbinafin, Nystatin, Amphotericin und/oder Undecylensäure.

Bevorzugt beträgt die Menge der erfindungsgemäßen wirksamen antimykotischen, antibakterioziden und/oder antibakteriostatischen Wirkstoffe in den Zubereitungen 0,0001 Gew.% bis 30 Gew.-%, vorzugsweise von 0,001 Gew.-% bis 15 Gew.%, insbesondere 0,1 Gew.% bis 10 Gew.% bezogen auf das Gesamtgewicht der Zubereitung. Die erfindungsgemäß wirksamen antibakteriostatischen und antibakterioziden Wirkstoffe F) können erfindungsgemäß mit einem oder mehreren Wirkstoffen von A) bis D) und G) bis H) in den dort angegebenen Konzentrationen kombiniert werden.

Erfindungsgemäß können in Kombination antivirale Wirkstoffe G) verwendet werden, wie z. B. Acyclovir, Famcyclovir, Gamcyclovir und deren Derivate.

Bevorzugt beträgt die Menge der erfindungsgemäßen wirksamen antiviralen Wirkstoffe G) in den Zubereitungen 0,0001 Gew.% bis 30 Gew.-%, vorzugsweise von 0,001 Gew.-% bis 15 Gew.%, insbesondere 0,1 Gew.% bis 10 Gew.% bezogen auf das Gesamtgewicht der Zubereitung. Die erfindungsgemäß wirksamen antiviralen Wirkstoffe G) können erfindungsgemäß mit einem oder mehreren Wirkstoffen von A) bis F) und von H) in den dort angegebenen Konzentrationen kombiniert werden.

Erfindungsgemäß können in Kombination bekannte, konventionelle Wirksysteme H) verwendet werden, wie z. B. Cyclosporin A, Cyclophilin, Rapamycin, FK 506, Corticoide und deren erfindungsgemäß wirksamen Derivate.
Bevorzugt beträgt die Menge der erfindungsgemäßen wirksamen konventionellen Wirkstoffe H) in den Zubereitungen 0,0001 Gew.% bis 30 Gew.-%, vorzugsweise von 0,001 Gew.-% bis 15 Gew.%, insbesondere 0,1 Gew.% bis 10 Gew.% bezogen auf das Gesamtgewicht der Zubereitung.

Die erfindungsgemäß wirksamen konventionellen Wirkstoffe H) können erfindungsgemäß mit einem oder mehreren Wirkstoffen von A) bis G) in den dort jeweils angegebenen Konzentrationen kombiniert werden.

Bevorzugt werden die folgenden Wirkstoffe und Wirkstoffkombinationen mit Stoffen aus A) bis H). Die dort angegebenen Gewichtsmengen sind besonders bevorzugte Gewichtsmengen und beziehen sich auf das Gesamtgewicht der Zubereitungen. Die Stoffe dienen, auch in den erfindungsgemäßen Zubereitungen, zur Behandlung der vorstehend angegebenen Hauterscheinungen:
1.Alpha - Glycosyl-Hesperidin (0.01 - 15 Gew. %), Ferulasäure (0,001 - 5 Gew. %), Biocytin (0,001 -0,8 Gew. %), Niacinsäure (0,001 -2,5 Gew. %, Thymol (0,005 - 2, 1 Gew. %), Vitamin E - acetat (0.002 - 12 Gew. %).
2. Naringin(0,002 - 10 Gew. % ),Ferulasäure (0,001 - 10 Gew. %), D-Biotin (0.01 - 0.4 Gew. %), Glucose-6-phosphat ( 0, 001 - 10 Gew. %), Vitamin E acetat (0,01 - 15 Gew. %), L-Carnosin (0,05 - 12 Gew.%), Ölsäure (0,05 - 0,5Gew.%), Harnstoff (0,01 - 12 Gew. %), Dextran(0,001 - 1 Gew. %), Neohesperidin( 0,005 - 3,0 Gew. %), Pyruvat (0,001 - 1,5 Gew. %).
3.Phloridzin (0,001 - 12 Gew. %), Cystamin (0,01 - 5,0 Gew.%), L-Histidin (0,01 - 6 Gew.%), β-Carotin (0,01 - 3 Gew. %), Ascorbylpalmitat (0,01 - 8,6 Gew. %), Oleylcystein (0,01 - 10 Gew. %), Gallenextrakt (0,02 - 2,2 Gew. %), ZnO (0,01 - 2,0 Gew. %), Folsäure (0,005 - 2,7 Gew. %), Rutinsäure (0.05-6,3 Gew.%), Harnsäure (0,02 - 1,0 Gew. %), Cyclosporin A (0,001 - 5.0 Gew. %), Ferulasäure (0,001 - 3,6 Gew. %), FMN (0,0005 - 0,25 Gew. %), Wollwachssäuren (0,005 - 2,5 Gew. %).
4. L - Anserin (0,01 - 3,2 Gew. %),Naringin (0.01 - 10 Gew. %),Hesperidin (0.01 - 12 Gew. %), Ferulasäure (0.001 - 9 Gew. %), Dextran(0,002 - 10 Gew. %), Butylhydroxyanisol (0,05 - 0,9 Gew. %), Heptadecensäure (0,01 - 0,9 Gew. %), N - Acetylcystein (0,01 -1,8 Gew.%),Huminsäure (0,01 - 2,7 Gew. %), ATP (0,004 -0,5 Gew. %), Biotinethylester (0.005 - 0,9 Gew.%),Kamillen-extrakt (0,001-6,7 Gew. %), L-Methionin (0,001 - 2,5 Gew. %), Oxothiazolidin-4-carboxylat (0,001-0,5 Gew.%).
5. Diosmin 0,01 -5 ,5 Gew. %, Lycopin (0,01 - 3, 8 Gew. %), L-Carnosin (0,01- 15 Gew. %), Glycin (0,02 - 1,2 Gew. %), Liponsäure (0,01 - 1,8 Gew. %), Folsäure (0,0008 - 0,5 Gew. %), Palmitinsäure (0,01 - 2,5 Gew. %), Mg-Ascorbylphosphat (0,002 - 5,4 Gew. %), Ethyl-cystein (0,004 - 4,5 Gew. %), Bilirubin (0,003 - 1,2 Gew. %), NADH (0,001 - 1,6 Gew. %), Mannose (0,01 -2,0 Gew. %, Grüner Tee-Extrakt (0.01-6,0 Gew. %), CaCl₂ (0,005-5,2 Gew. %), Biocytin (0,005 - 0,8 Gew. %), Catalase 0.001 - 1.0 Gew. %).
6. Katzenpfötchenblüten - Extrakt(0,01 - 10,0 Gew. %), ZnO (0,02 - 1,4 Gew. %), Selen-methionin (0,01 - 0,8 Gew. %), Ferulasäure (0,02 - 4,2 Gew. %), Sophora Japonica - Extrakt(0,01 - 7,9 Gew. ),Salicylsäure(0,01 - 3,6 Gew. %), Ubiquinol (0,003 - 2,8 Gew. %), Vitamin E palmitat (0,02 - 8.6 Gew. %), Vitamin A -oleat (0,02 - 2,0 Gew. %), Konyferylbenzoat (0,05 -1,5 Gew. %), Liposomen (0,2 - 2,5 Gew. % ), GTP (0,001 - 0,9 Gew. %), gamma-Linolensäure aus Pflanzenölen (0,01 - 15 Gew. %), Kaffee - Extrakt (0,01-5,8 Gew. %), Dimethylisosorbit (0,02 - 3,4 Gew. %),CaCl₂ ( 0,005 - 3,5 Gew. %), MgCl₂ (0,01 - 2,8 Gew. %).
7. Cis- Urocaninsäure (0,005 - 2,0 Gew. %), Zitrusfruchtschalenextrakt0.1 - 8 Gew.%, Phytinsäure (0,01 - 2,7 Gew. %), alpha-Carotin (0,05 - 2,9 Gew.%), Thioglucose (0,001 - 0,9 Gew. %), Ascorbylglucoside (0,08 - 5,8 Gew. %), Linolsäure (0,05 - 6,4 Gew.%), Lactoferrin (0.002 - 1,0 Gew. %), Dilaurylthio-dipropionat (0,2 - 2,0 Gew. %), Ubiquinon (0,001 - 2,1 Gew. %), ZnSO₄ (0,001 - 2.0 Gew. %), Glutathion (GSSG) 0,004 - 4,1 Gew.%, Gallensäureextrakt(0,002 - 2,5 Gew. %).
8. Ein oder mehrere oxidierte und/oder reduzierte Thiole (0,001 - 10 Gew. %) aus der Gruppe der antioxidativen Wirkstoffe, wie z. B.reduziertes Glutathion (GSH), oxidiertes Glutathion (GSSG), Cystein, N-Acetylcystein,Cystein-S-acetat,Cystin, Oxothiazolidin-4-carboxylat und deren Ester (z. B. Methyl-, Ethyl-, Butyl-, Propyl-, Amyl-, Sorbitosyl-, Galactosyl-, Mannosyl-, Glucosyl-, Glycosyl-, Acetyl-, Lauryl-, Palmitoyl-, Oleyl-, Linoleyl-, Cholesterylester) und deren Salze (z. B. Distearylthiopropionat, Dioleylcysteinylpropionat) in Kombination mit einem oder mehreren Wirkstoffen der unter 1. - 7. beschriebene Wirksystemen.
9. Erfindungsgemäß wirksame Peptid- Verbindungen (0,001 - 14 Gew. %) und/oder deren Verbindungen aus der Gruppe der antioxidativen Wirkstoffe, insbesondere mit Di - und/oder Tripeptidverbindungen, die die Aminosäuren Histidin, Tryptophan, Tyrosin, Methionin, Glutaminsäure enthalten (z. B. L-Alanylhstidin, L-Glycylhistidin, L-Histidyltryptophan , L-Histidylmethionin, Se-Methionylhistidin) und/oder Polyamine wie z. B. Spermin, Spermidin in Kombinationen mit einem oder mehreren Wirkstoffen der erfindungsgemäß genannten Wirksysteme 1 - 8.
10. Eine oder mehrere erfindungsgemäß wirksame Fettsäuren und Lipidverbindungen (0,0005 - 12 Gew. %) und/oder deren Verbindungen aus der Gruppe der Energiestoffwechselregulatoren mit den unter 1 - 9 genannten Wirkstoffen (z. B. Lauryl-, Oleyl-, Linoteyl-, Palmitoyl-, Stearyl- und Cholesteryl-, Furan -Verbindungen) in Kombinationen mit einem oder mehreren der genannten Wirksysteme 1 - 9.
11. Eine oder mehrere erfindungsgemäß wirksame Nuleinsäure - Derivate, Nukleosid und Nukleotidverbindungen (0,0001 - 7 Gew. %) und deren Verbindungen aus der Gruppe der Energiestoffwechselregulatoren (z. B. Propylthiouracil, Ethylthioadenosin, Isopropylguanosylcystein) in Kombination mit einem oder meheren Wirkstoffen der erfindungsgemäß genannten Wirksysteme 1 - 10.
12. Ein oder mehrere erfindungsgemäß wirksame enzymatische Antioxidanssysteme (0,0001 - 5 Gew. %) und deren synthetische Mimics wie z. B. die Enzyme Superoxiddismutase, Glutathionperoxidase, Glutathionreductase, Glutathion-S-Transferase, Oxidoreductasen (z. B. Glucose-6-phosphat - dehydrogenase, Succinatdehydrogenase, Malatdehydro-genase, insbesondere die NAD⁺-, NADP⁺- und FAD⁺-Reduktasen sowie Carboxylasen (z. B. Biotin-carboxylase, Acetyl-CoA Carboxylase), Cytochrom P 450- Reductase, Cytochrom P450 - Monooxigenasen, Monoaminoxidase in Kombinationen mit einem oder mehreren Wirkstoffen der unter 1-11 genannten Wirksysteme.
13. Ein oder mehrere erfindungsgemäß wirksame Coenzymen (0,0001 - 5 Gew. %) und deren Vorstufen (z. B. Provitamine, Vitamine) sind z. B. Biocytin, D-Biotin und dessen Derivate (z. B. Biotinethyl, -methyl, butyl-, -propionyl, -isopro-pionylester, Biocytin), Glucose und Glucose-Derivate (z.B. Glucose-6-phosphat, Glucose-1,6-phosphat, Glucosylcystein, Glucosylcystin, Dextrane, Glycosyl-cysteine, Glycosylcystine, Glucosylgluthation, Glucosylcystamin, Glycosylcystamin), Pyruvat, Coenzym A und dessen Derivate, Niacinsäure, NADH, NADPH, Adenin, Adenosin,Methyl-S-adenosin, AMP, cAMP, ADP, ATP, Guanin, Guanosin, GMP, cGMP, GDP, GTP und FAD⁺, FADH₂, FMN, Folsäure, Dihydrofolat, Riboflavine, Panthotensäure, Panthenol, Thiaminpyrophosphat, Thiamin, 6 - Phosphoglucurono -delta- lacton, 6 - Phosphoglucon-säure, Fructose- 6-phosphat, Glycerinaldehyd - 3-phosphat, Ribulose - 5 - phosphat, Pyridoxamin, Pyridoxalphosphat, Biopterine (z. B. Aminopterin, Tetrahydropterin), Coenzym Q und dessen Derivate (z. B. Ubiquinol), Vitamin D und deren Derivate (z. B. Vitamin D₃) und die geeignete Derivate (Salze, Zucker, Ester, Alkohole, Ether, Nukleotide, Nukleoside, Peptide und Lipide) in Kombinationen mit einem oder mehreren Wirkstoffen der unter 1-12 genannten Wirksysteme.
14. Flavonoide , z. B. Alpha-Glycosylrutin und/oder Hesperidin und/oder Naringin als Einzelsubstanz oder in Kombinationen mit einem oder mehreren Wirkstoffen der unter 1-13 genannten Wirksysteme.
15.Flavonoide in Kombination mit Zimtsäurederivaten, z. B. Ferulasäure und/oder Kaffeesäure, in Kombination mit einem oder mehreren Wirkstoffen der unter 1-14 genannten Wirksysteme.

Diese Einzelkomponenten in den jeweiligen Wirksystemen 1 - 15 und deren Konzentrationsangaben sind beispielhaft genannt, ohne damit die Verwendung anderer genannter Wirkstoffe und deren Derivate oder deren erfindungsgemäß wirksamen Kombinationen und Konzentrationen ausschließen zu wollen.

Es erwies sich ferner als vorteilhaft bei der Prophylaxe und Behandlung der erfindungsgemäß unter I. bis XIII. benannten Dermatosen und hyperreaktiver und/oder hypoaktiver, defizitärer Hautzustände, die erfindungsgemäßen Wirkstoffe und insbesondere die Wirkstoffsysteme 1 - 15 mit Harnstoff und/oder Arginin (z. B. 0,01 - 15 Gew. %) zu kombinieren. Die Verwendung von Harnstoff in kosmetischen und dermatologischen Formulierungen zur Behandlung, insbesondere von trockenen Hautzuständen, ist allgemeiner Stand der Technik.

Unter dem Begriff hypoaktive Hautzustände sind erfindungsgemäß degenerative Hautzustände zu verstehen, die eine Unterfunktion der Haut und der Hautanhangsgebilde zur Folge haben. Diese Unterfunktion kann seine Ursachen auf molekularer Ebene, intra - oder extrazellulär, haben , wie z. B. eine Unterfunktion des Glutathionredoxsystems, der Catalase, der Superoxiddismutase, oder der Pyruvatcarboxylase. Als Konsequenz wäre der intra - und extrazelluläre Antioxidansstatus oder Energiestoffwechselstatus einzelner Zellen, z. B. der Fibroblasten in der Dermis oder der Keratinozyten und Langerhanszellen in der Epidermis oder auch der gesamten Haut hypoaktiv.

Unter dem Begriff hyperaktive Hautzustände sind erfindungsgemäß Hautzuständ zu verstehen, bei denen eine über das normale Niveau hinausgehende Überexpression z. B. von einzelnen Stoffwechselwegen oder Enzymaktivitäten ,wie z. B. Phoshodiesteraseaktivität, Monoaminoxigenaseaktivität, Transaminaseaktivität, vorliegt. Als Konsequenz kann eine Hyperproliferation z. B. der Keratinozyten oder eine Hyperaktivität von Langerhanszellen oder T-Lymphozyten gefunden werden.
Unter den erfindungsgemäß genannten manifesten Dermatiden sind die unter I. bis XIII. benannten Dermatiden und deren Ausprägungsformen und Synonyme gemeint.

Es erwies sich auch als vorteilhaft, für die Prophylaxe und Behandlung der erfindungsgemäß unter I. bis XIII. genannten Dermatiden sowie für die hypo- und hyperaktiven Hautzustände die Wirkstoffe und Wirkstoffkombinationen A) bis E) zu verwenden.

Für die Behandlung und Prophylaxe der mikrobiell assoziierten Dermatiden, wie z. B. Atopisches Ekzem, Psoriasis-, Akne-Formen, Seborrhoischem Ekzem, Ichthyosis und bakterielle Infektionen und Mykosen, wie z. B. bei der Windeldermatitis, erwies es sich von Vorteil die erfindungsgemäßen Wirkstoffe und Wirkstoffkombinationen A) bis E) und H) mit den antimikrobiellen Wirksystemen F) zu kombinieren.

Für die Behandlung und Prophylaxe der viral assoziierten Dermatiden, wie z. B. die erfindungsgemäß unter IV: genannten Herpeslnfektionen erwies es sich von Vorteil die erfindungsgemäßen Wirkstoffe und Wirkstoffkombinationen A) bis E) und H) mit den antimikrobiellen Wirksystemen G) zu kombinieren. Bei viralen und mikrobiellen Superinfektionen erwies es sich insbesondere von Vorteil, den gewählten Wirkstoffkombinationen aus A) bis D) und H) auch eine Kombination von Wirkstoffen aus F) und G) hinzuzufügen.

Von besonderemVorteil sind bei der Prophylaxe und Behandlung von hypo- und hyperaktiven Hautzuständen und den erfindungsgemäß genannten Dermatosen und defizitären Hautzustände folgende Kombinationen A - G zu verwenden, die insbesondere für:
Atopischen Dermatiden z.B. aus folgenden Einzelsubstanzen und deren Derivaten zusammengesetzt sein können:
Kombination A: Alpha - Glycosylrutin 0,4 Gew. %, Hesperidin 0.1 Gew. %,L- Carnosin 0,4 Gew. %,D-Biotin 0.05 Gew. %, Vitamin A- palmitat 0,8 Gew. %, Vitamin E - acetat 1,5 Gew. %, Wollwachssäuren 0,2Gew.%, Zitronensäure 1,0 Gew. %, Glucosylcystamin 0.04 Gew.%, Ölsäure 0,3 Gew. %, Adenosin 0,8 Gew. %, Ferulasäure 1,2 Gew. %, Ubiquinon 0,5 Gew. %,Vanilinsäure 0,2 Gew.%.

Von besonderem Vorteil erwiesen sich bei diesen Dermatosen auch Kombinationen dieser Wirkstoff-Zusammensetzung mit den obengenannten Wirksystemen 1 - 4, 7 - 9, 12 und 13.

Ferner erwies sich die Kombination der genannten erfindungsgemäßen Einzelwirkstoffe und der unter 1 - 13 und A - F genannten Wirksysteme mit Harnstoff als vorteilhaft.

Weiterhin bevorzugt wird, insbesondere für **Psoriasis**, z.B. die folgende Kombination B, die z.B. aus folgenden Einzelsubstanzen und deren Derivaten zusammengesetzt sein kann:
Kombination B: Alpha-Glycosyl-Naringin 2,8 Gew. %, Carnosin 1,0 Gew. %, Methyl- S - Adenosin 1.5 Gew.%, Ferulasäure 0,4 Gew.%,alpha - Hydroxypalmitinsäure 0,2 Gew.%, Ölsäure 0,3 Gew. %, Cystein-S-acetat 0,05 Gew. %, Vitamin E -acetat 3,0 Gew. %, Vitamin A -palmitat 1,0 Gew.%, Deoxycholat 0,04 Gew. %.

Von besonderem Vorteil erwies sich bei Psoriasis-Formen auch die Behandlung mit diesen Wirkstoffen in Kombination mit den Wirksystemen 2 - 5,7,8,10 und 13.

Weiterhin bevorzugt wird, insbesondere für **Prurigo-Formen und Pruritus-Formen**, z.B. die folgende Kombination C, die aus folgenden Einzelsubstanzen und deren Derivaten zusammengesetzt sein kann:
Kombination C: Phloridzin 1,8 Gew.%, Ferulasäure 0,8 Gew. %, Diosmin 0,2 Gew %, 0,8 Gew. %, Zitronensäure 1,4 Gew.%, Glutamylcystein 0,05 Gew.%, Glutaminsäure 0,5 Gew. %, L - Arginin 1,0 Gew.%, Vitamin E-palmitat 1,5 Gew. %, Liponsäure 0,005 Gew. %, L-Carnosin 1,8 Gew. %,Sylimarin 0,3 Gew. % .

Von besonderem Vorteil erwiesen sich zur Behandlung von Prurigo-Formen und Pruritus-Formen auch diese Wirkstoffe in Kombination mit den Wirksystemen 2 - 4, 6,7,9,12 und13.

Weiterhin bevorzugt wird, insbesondere für **Photodermatosen, vor allem bei Polymorpher Lichtdermatose**, z.B. die folgende Kombination D, die aus folgenden Einzelsubstanzen und deren Derivaten zusammengesetzt sein kann:
Kombination D: Glycosylhesperidin 2,0 Gew. %,alpha Glycosylrutin o,3 Gew. %, Ferulasäure 1,2 Gew. %, D,L-Carnosin 0,7 Gew.-%, L-Tyrosin 0, 2 Gew. %,Methionin 0,9 Gew. %,Vitamin E -acetat 2,0 Gew. %, Mannose 0,5 Gew. %, Äpfelsäure 1,0 Gew. %, Ölsäure 0,3 Gew. %, Harnsäure 0,05 Gew.%, Cystein-S-acetat 0,8 Gew. %, Ascorbylpalmitat 1,5 Gew.-%.

Von besonderem Vorteil erwiesen sich zur Prophylaxe und Behandlung von Photodermatosen, insbesondere von Polymorpher Lichtdermatose, auch die Kombinationen dieser Wirkstoffe mit den Wirksystemen 3, 5 - 9 und 12.

Ferner wird bevorzugt, insbesondere zur Prophylaxe und Behandlung von **Akneformen**, die Kombination E, die aus folgenden Einzelsubstanzen und deren Derivaten zusammengesetzt sein kann:
Kombination E: Naringinin 2,8 Gew. %,Z-Ferulasäure 1,2 Gew.%, Vanilinsäure 0,3 Gew. %, Methionin 0,6 Gew. %, L-Serin 0,9 Gew. %, D-Biotin 0,02 Gew. %, Zitronensäure 0,5 Gew. %, Milchsäure 0,4 Gew. %, Apfelsäure 0,3 Gew. %, Kaffesäure 0.9 Gew. %, Glutaminsäure 0,5 Gew. %, Panthotensäure 0,6 Gew. %, Vitamin E -nicotinat 0,5 Gew. %, Salicylsäure 0,8 Gew. %, Gerbsäure 0,3 Gew. %, Thiamin 0.3 Gew. %, Ölsäure 0,3 Gew. %.

Von besonderem Vorteil erwiesen sich zur Prophylaxe und Behandlung auch die Kombinationen dieser Wirkstoffe mit den Wirksystemen 3, 6, 7, 9, 12 und 13.

Für die Prophylaxe und Behandlung der genannten viralen **Infektione**n, insbesondere von Herpesinfektionen der Schleimhäute, erwies sich die Kombination F, die z. B. aus folgenden Einzelsubstanzen zusammengesetzt sein kann, als besonders geeignet:
Kombination F: Glucosyl-Naringin 2,0 Gew. %,E - Ferulasäure 0,5 Gew. %, Biocytin 0,15 Gew. %, L-Methionin 1,1 Gew. %, Niacinsäure 0,3 Gew %, Gerbsäure 0,4 Gew. %, Zitronensäure 0,4 Gew. %, AE - Complex/Fa. Solabia 1,5 Gew.%, β -Glucan 1,5 Gew. %, Soyaextrakt 0,3 Gew. %.

Von besonderem Vorteil erwiesen sich zur Prophylaxe und Behandlung von viralen Infektionen auch die Kombi-nationen dieser Wirkstoffe mit den Wirksystemen 1, 4 - 12.

Für die Prophylaxe und Behandlung von Wunden, insbesondere von chronischen Wunden, wie z. B. **Ulcus cruris** oder **Decubitus** erwies sich die Kombination G, die aus folgenden Einzelsubstanzen bestehen kann, als besonders geeignet.

Kombination G: Naringinin 0,6 Gew. %, Diosmin 0,5 Gew. %, alpha-Glycosylhesperitin 0,8 Gew. %, Ferulasäure 0,2 Gew. %,Biotin 0,05 Gew. %, Zitronensäure 0,4 Gew. %, Dextran (MW 10 000 - 5 Mio.) 2 Gew. %,Folsäure 0.2 Gew.%, Niacinsäure 0.4 Gew. %, Katzenpfötchenblütenextrakt 0,5 Gew. %.

Von besonderem Vorteil erwiesen sich zur Prophylaxe und Behandlung insbesondere von chronischen Wunden auch die Kombinationen dieser Wirkstoffe mit den Wirksystemen 1, 4 -13.
Diese Einzelkomponenten und deren Konzentrationen in einer Zubereitung sind beispielhaft genannt, ohne damit die Verwendung anderer Andioxidantien und Wirkstoffe auszuschließen.

In den vorstehenden Kombinationen A bis G beziehen sich die angegebenen Gew.-%-Angaben jeweils auf das Gesamtgewicht der Zubereitungen oder z.B. der Zubereitungen der Beispiele. In den Beispielen werden diese Kombinationen A bis G verwendet.

Zur Prophylaxe werden die Wirkstoffe verabreicht, um Manifestationen der Krankheiten in der Häufigkeit und Stärke zu mindern. Die Behandlung im manifesten Stadium führt zu deren Verkürzung und zur Milderung der Symptome.

Ferner dient die Behandlung mit den erfindungsgemäßen Wirkstoffen zur Vorbeugung oder Milderung von Nebenwirkungen und Folgeschäden, die durch Anwendung herkömmlicher Therapieformen, wie z. B. der UV - Therapie von Psoriasisformen (PUVA - Therapie)sowie von Atopischem Ekzem und Lichtdermatosen, auftreten können.
Außerdem erlauben die erfindungsgemäßen Wirksysteme als begleitende Therapieform eine Reduktion in der Dosierung von herkömmlichen, pharmakologischen Wirkstoffen, wie z. B. Corticoide, bei entzündlichen Dermatosen.

Ferner ist es von Vorteil, die erfindungsgemäßen Wirkstoffe und Zubereitungen, auch in Kombination mit herkömmlichen Wirkstoffen und Methoden zu verwenden, die zur Behandlung der genannten Dermatosen üblicher-weise verwendet werden (z.B. Cyclosporin A, Corticosteroide, Psoralen, UV-Therapie,PUVA-Therapie, UVA und /oder UVB-Filter, Harnstoff, Antibiotika, Acyclovir).

Die erfindungsgemäßen Wirkstoffe und deren Kombination und die damit erhaltenen Zubereitungen wie pharma-zeutische Präparate und topische Zubereitungen in Form von Cremes, Lotionen, Gelen und Sprays sowie Lösungen (z.B. wäßrige, alkoholische oder wäßrig-alkoholische) und anderen geeignete Formulierungen sind insbesondere prophylaktisch wirksam, indem sie empfindliche, für die unter I. bis XIII. benannten Dermatosen prädisponierte Haut, insbesondere bei Atopischen Dermatiden und Lichtdermatosen, schützen oder deren Ausbildung mindern. Dazu werden die wirkstoffhaltigen Formulierungen einmalig und mehrmals täglich regelmäßig angewandt. Bei den benannten manifesten Dermatosen, insbe-sondere bei atopischen Dermatiden (z.B. atopischem Ekzem, Neurodermitis), Ichthyosis, Polymorphe Lichtdermatose, Psoriasis und Pruritus erfolgt nach hinreichender Behandlung mit den erfindungsgemäßen Wirk-stoffen und Zubereitungen eine Besserung der Hautzu-stände, insbesondere ein Abklingen des bei diesen Dermatosen auftretenden Juckreizes. Anschließend kann prophylaktisch weiterbehandelt werden.

Es war überraschend und auch für den Fachmann nicht vorhersehbar, daß die erfindungsgemäßen Wirkstoffe und Zubereitungen, insbesondere nach topischer Applikation auf die menschliche Haut, zur Prophylaxe und Behandlung der unter I. bis XIII. genannten Dermatosen und der empfindlichen, hyperreaktiven und für Dermatiden prädisponierten Haut dienen können und die erfindungsgemäßen Kombinationen von Flavonglycosiden und Zimtsäurederivaten und/oder mit Antioxydanssystemen und/oder Wirkstoffen, die den endogenen Energiestoffwechsel, insbesondere in der Haut, und/oder antimikrobiellen Wirkprinzipien eine synergistische Wirkung erreichen können.

Der Wirkmechanismus der neuen, erfindungsgemäßen Wirk-stoffe und Zubereitungen ist noch nicht vollständig geklärt. Es wird angenommen, daß bei den betreffenden defizitären oder pathologischen Hautzuständen ein erhöhter oxidativer, radikalischer Streß als Ursache vorliegt, der durch die Behandlung mit den erfindungsgemäßen Wirkstoffen gelindert oder gar auf das normale Niveau intakter und gesunder Haut reguliert wird. Vorteilhaft ist auch, daß die erfindungsgemäß einge-setzten Wirkstoffe auch die enzymatischen Antioxidans- und Reparatursysteme sowie den endogenen Energiestoff-wechsel der Haut unterstützen, so daß ein additiver oder gar synergistischer Wirkmechanismus bei den erfindungsgemäßen verwendeten Wirksystemen und Zube-reitungen vorliegt. Diese Wirkstoffe können an der Oberfläche der Haut, Schleimhaut und/oder Hautanhangsgebilde beziehungsweise durch Penetration über die Talg- und Schweißdrüsen, aber auch entlang von Haarschäften oder durch das Stratum corneum an den Wirkort gelangen. Dazu können einerseits bestimmte erfindungsgemäße Wirkstoffe (z. B. antimikrobielle Wirkstoffe) in den erfindungsgemäßen Zubereitungen z. B. durch Kopplung an spezifische Träger an der Pene-tration in die Haut gehindert werden, so daß nur eine oberflächliche Wirkung auf der Haut und/oder den Haaren und/oder in den Talg- und/oder Schweißdrüsen und/oder den Haarschäften erzielt wird. Für eine Verbesserung der Penetration von bestimmten Wirkstoffen in die Haut können andererseits Penetrationsförderer wie z. B. DMSO, Azone, ungesättigte Fettsäuren (z. B. Ölsäure, cis-Alkenfettsäuren), Heptadecensäure, Liposomen, Nanosomen, Mikrosomen, Niosomen und andere Verkapse-lungsformen zugefügt werden bzw. die Wirkstoffe in diesen verpackt werden, um einen Retard- und/oder Konservierungseffekt zu erreichen.

Ferner können insbesondere zur Prophylaxe und Behandlung von Dermatiden wie z. B. Neurodermitis, Atopisches Ekzem, Seborroisches Ekzem, Rosacea, Ichthyosis, Akne- und Psoriasisformen sowie von viralen Infektionen wie z. B. Herpes simplex, H. labialis und H. zoster antimikrobielle und /oder antivirale Wirkprintipien in Kombination mit den erfindungsgemäßen Antioxydans- und / oder den endogenen Energiestoffwechsel regulierenden Systemen verwendet werden.

Ferner sollten solche Wirksysteme bzw. Zubereitungen Wirkstoffe enthalten, welche zur Immunhomeostase der kranken Haut, dabei vorteilhaft auch zur Immunstimulation im Sinne der die Wundheilung fördernden Wirkung, insbesondere bei Decubitus und Ulcus cruris, verwendet werden können.

Insbesondere vorteilhaft werden die erfindungsgemäßen Wirkstoffe gewählt aus der Gruppe der Flavonoide und ihrer Glucoside, aus der Gruppe der Zimtsäurederivate, insbesondere aus der Gruppe der Ferulasäure - und Kaffeesäure- Derivate sowie aus der Gruppe der Antioxidantien, insbesondere der Gruppe der Tocopherole und ihrer Derivate und der Imidazole und deren Derivate.

Die Schrift JP-OS Hei-06-138,941 beschreibt zwar orale Zubereitungen mit einem Gehalt an wasserllöslichen Glucosiden, welche beispielsweise gewählt werden können aus der Gruppe α-Glucosylrutin, α-Glucosylmyrictrin, α-Glucosylisoquercitrin und α-Glucosylquercitrin. Die Schrift JP-OS Hei-04-363,395 beschreibt ein Verfahren, die Zersetzung von Parfümbestandteilen zu verhindern, welche sich unter anderem durch einen Zusatz an α-Glucosylrutin zu den entsprechenden Zubereitungen auszeichnet. Ferner beschreiben die Schriften EP-OS 586 303 und EP-OS 595 694 die Verwendung von Flavonoïden als Antioxidantien bzw. Licht-schutzsubstanzen in Kosmetika. Weiterhin ist aus US-PS 4,144,325 und 4,248,861 sowie aus zahlreichen anderen Dokumenten bekannt, Vitamin E in kosmetischen und dermatologischen Lichtschutzformulierungen einzusetzen. Die erfindungsgemäße Verwendung des Vitamins E und seiner Derivate in den erfindungsgemäßen Kombinationen zur kosmetischen oder dermatologischen Prophylaxe und/oder Behandlung der genannten Dermatosen sowie der hyper- und hypoaktiven Hautzustände wurde indes nicht durch den Stand der Technik nahegelegt.
Kein Hinweis ist diesen Schriften zu entnehmen, welcher in die Richtung der vorliegenden Erfindung weisen könnte.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die erfindungsgemäßen kosmetischen und/oder dermatologischen Formulierungen können wie üblich zusammengesetzt sein und zur Prophylaxe und/oder zur Behandlung der Haut im Sinne einer dermatologischen Behandlung oder einer Prophylaxe und/oder Behandlung im Sinne der Kosmetik dienen. Sie enthalten bevorzugt 0,01 Gew.-% bis 20 Gew.-%, insbesondere aber 0,1 Gew.-% bis 6 Gew.-%, bezogen auf das Gesamtgewicht eines oder mehrerer erfindungsgemäßer Wirkstoffe.

Es ist erfindungsgemäß vorteilhaft, Kombinationen aus mehreren der erfindungsgemäßen Wirkstoffenzu verwenden, insbesondere, wenn wenigstens eine der Komponenten, gewählt wird aus der Gruppe der Flavonoide bzw. deren Glucoside und der Zimtsäurederivate, ausgewählt wird.

Vorteilhaft ist es insbesondere, Kombinationen aus Flavonoiden bzw. deren Derivaten, Zimtsäure und ihren Derivaten sowie Antioxidantien,insbesondere Vitamin E bzw. seinen Derivate, zu verwenden.

Zur bevorzugten Anwendung werden die erfindungsgemäßen Wirksysteme bzw. Zubereitungen, solche Wirkstoffe enthaltend, vorzugsweise Kombinationen aus Flavonoiden bzw. deren Derivaten, Zimtsäure und deren Derivaten sowie Antioxidantien, insbesondere Vitamin E und dessen Derivate in Kombination mit Energiestoffwechselmetaboliten und/oder antimikrobiellen Wirksystemen in der für Kosmetika oder Dermatika üblichen Weise auf die Haut in ausreichender Menge aufgebracht.

Die erfindungsgemäßen kosmetischen und dermatologische Zubereitungen können in verschiedenen Formen vorliegen. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), Öl-in-Wasser-in-Öl (O/W/O), ein Gel, einen festen Stift oder auch ein Aerosol darstellen. Die Herstellung erfolgt in an sich bekannter Weise.
Die erfindungsgemäßen kosmetischen und dermatologische Zubereitungen können kosmetische und dermatologische Hilfsstoffe enthalten, wie sie üblicherweise in solchen Zubereitungen verwendet werden, z.B. UV/A- und UV/B- Filter, Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaum-stabilisatoren, Elektrolyte, organische Lösungsmittel oder Silikonderivate.

Es ist auch vorteilhaft, die erfindungsgemäßen Wirkstoffkombinationen in verkapselter Form darzureichen, z.B. in Kollagenmatrices und anderen üblichen Verkapselungsmaterialien,z.B.alsCelluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, oder flüssige Triglceride natülicher Herkunft
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Wasserfreie kosmetische und dermatologische Zubereitun-gen wie Salben oder Hautöle gemäß der Erfindung sind vorteilhaft und enthalten z. B. die genannten Fette, Öle, Silikonöle, Wachse und anderen Fettkörper.

Feste Stifte gemäß der Erfindung enthalten z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.
Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise
   Salicylsäure(2-ethylhexyl)ester,
   Salicylsäure(4-isopropylbenzyl)ester,
   Salicylsäurehomomenthylester;
- Derivate des Benzophenons, vorzugsweise
   2-Hydroxy-4-methoxybenzophenon,
   2-Hydroxy-4-methoxy-4'-methylbenzophenon,
   2,2'-Dihydroxy-4-methoxybenzophenon;
- Ester der Benzalmalonsäure, vorzugsweise
   4-Methoxybenzalmalonsäuredi(2-ethylhexyl)ester;
- 2,4,6-Trianilino-(p-carbo-2'-ethyl-1'-hexyloxy) -1,3,5-triazin.

Als wasserlösliche Substanzen sind z.B. zu nennen:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise
   2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Radikalfängern verwendet werden können, soll selbstverständlich nicht limitierend sein.

Gegenstand der Erfindung ist auch die Kombination eines oder mehrerer erfindungsgemäßer Wirkstoffe mit einem oder mehreren UVB-Filtern bzw. erfindungsgemäße kosmetische oder dermatologische Zubereitungen, welches auch einen oder mehrere UVB-Filter enthalten.

Es kann auch von Vorteil sein, einen oder mehrere erfindungsgemäße Wirkstoffe mit UVA-Filtern zu kombinieren, die bisher üblicherweise in kosmetischen und/oder dermatologischen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner werden vorteilhafte Zubereitungen erhalten, wenn die erfindungsgemäßen Wirkstoffen mit UVA- und UVB-Filtern kombiniert werden.

Kosmetische Zubereitungen, erfindungsgemäße Radikal-fänger enthaltend, können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon sowie Abwandlungen, bei denen die Oxide die aktiven Agentien sind. Besonders bevor-zugt handelt es sich um Pigmente auf der Basis von Titandioxid.

Auch diese Kombinationen von UVA-Filter und/oder UVB-Filter und Pigment bzw. Zubereitungen, die diese Kombination enthalten, sind Gegenstand der Erfindung. Es können die für die vorstehenden Kombinationen genannten Mengen verwendet werden.

Als vorteilhafte Verkörperung der vorliegenden Erfindung wird daher auch die Verwendung erfindungsgemäßer Wirkstoffkombinationen zum Schutze der Haut und/oder der Haare vor oxidativer Beanspruchung angesehen, insbesondere diese Verwendung der erfindungsgemäßen Wirkstoffkombinationen in Shampoos und Waschformulierungen. Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, erfindungsgemäße Wirkstoffe und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegan.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nicht-ionische oder kationische oberflächenaktive Substanzen, deren Konzentration zwischen 0,1 und 10 Gew.-%, vorzugsweise zwischen 0,2 und 5 Gew.-%, liegen kann. Diese kosmetischen oder dermatologischen Zubereitungen können auch Aerosole mit den üblicherweise dafür verwendeten Hitfsmitteln darstellen.

Zubereitungen zur Behandlung der Kopfhaut und Haare, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.
Erfindungsgemäß können kosmetische Zubereitungen zur Behandlung der Haut, Kopfhaut und Haare als Gele oder Haarwässer vorliegen, die neben einem wirksamen Gehalt an erfindungsgemäßen Wirkstoffen und dafür üblicher-weise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispiels-weise Bentonite, oder ein Gemisch aus Polyethylenglykol und Polyethylenglycolstearat oder - distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Alle Mengenangaben, Anteile und Prozentanteile sind, soweit nicht anders angegeben, auf das Gewicht und die Gesamtmenge bzw. auf das Gesamtgewicht der Zubereitungen bezogen.
Gemäß der Erfindung werden als Zubereitungen auch pharmazeutische Präparate, Mittel oder Zusammensetzungen geschaffen, die die erfindungsgemäßen Verbindungen oder deren Derivate oder pharmazeutisch verträgliche Salze zusammen mit einem pharmazeutisch verträglichen Verdünnungsmittel oder Träger enthalten.

Die Verbindungen der vorliegenden Erfindung können beim Menschen oral oder parenteral z.B. in einer Dosierung von 0,01 bis 5000 mg, vorzugsweise 1 bis 500 mg, pro Tag angewendet werden, insbesondere auch in unterteilten Dosen, zum Beispiel zweimal bis viermal täglich.

Die Wirkstoffe gemäß der Erfindung können mit üblichen pharmazeutisch verträglichen Verdünnungsmitteln oder Trägern und gegebenenfalls mit anderen Hilfsmitteln vermischt und beispielsweise oral oder parenteral verabreicht werden. Sie können vorzugsweise oral in Form von Granulaten, Kapseln, Pillen, Tabletten, Filmtabletten, Dragees, Sirupen, Emulsionen, Suspensionen, Dispersionen, Aerosolen und Lösungen sowie Flüssigkeiten, oder aber auch als Zäpfchen, Vaginalkugeln oder parenteral z.B. in Form von Lösungen, Emulsionen oder Suspensionen verabreicht werden. Oral zu verabreichende Präparate können einen oder mehrere Zusätze wie Süßungsmittel, Aromatisierungsmittel, Farbstoffe und Konservierungsmittel enthalten. Tabletten können den Wirkstoff mit üblichen pharmazeutisch verträglichen Hitfsmitteln vermischt enthalten, zum Beispiel inerten Verdünnungsmitteln wie Calciumcarbonat, Natriumcarbonat, Lactose und Talk, Granulierungsmitteln und Mitteln, die den Zerfall der Tabletten bei oraler Verabreichung fördern wie Stärke oder Alginsäure, Bindemitteln wie Stärke oder Gelatine, Gleitmitteln wie Magnesiumstearat, Stearinsäure und Talk.

Geeignete Trägerstoffe sind beispielsweise Milchzucker (Lactose), Gelatine, Maisstärke, Stearinsäure, Ethanol, Prophylenglycol, Ether des Tetrahydrofurfurylalkohols und Wasser.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calziumcarbonat und Dicalziumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Kapseln können den Wirkstoff als einzigen Bestandteil oder vermischt mit einem festen Verdünnungsmittel wie Calciumcarbonat, Calciumphosphat oder Kaolin enthalten. Die injizierbaren Präparate werden ebenfalls in an sich bekannter Weise formuliert.

Die pharmazeutischen Präparate können den Wirkstoff in einer Menge von 0,1 bis 90 Gewichtsprozent, insbesondere 1-90 Gew.-% enthalten. Kapseln werden besonders bevorzugt Einzeldosen enthalten die Wirkstoffe vorzugsweise in einer Menge von 0,01 mg - 500 mg.

Gegenstand der Erfindung sind auch pharmazeutische und topische Zubereitungen, die dadurch gekennzeichnet sind, daß sie eine Kombination von erfindungsgemäßen Antioxydantien und regulierenden Wirkstoffen enthalten sowie die Kombination von erfindungsgemäßen Antioxydantien und/oder regulierenden Wirkstoffen mit antiviralen und/oder antimikrobiellen Wirkprinzipien wie vorstehend beschrieben.

Alle Mengenangaben, Anteile und Prozentanteile sind, soweit nicht anders angegeben, auf das Gewicht und die Gesamtmenge bzw. auf das Gesamtgewicht der Zubereitungen bezogen.
In den Beispielen werden auch die genannten Kombina-tionen A - G von Einzelsubstanzen mit den angegebenen Gewichtsteilen verwendet, die jeweils die Gewichtsverhältnisse der Einzelsubstanzen in der Zubereitung wiedergeben.

Die nachfolgenden Beispiele sollen die vorliegende Erfindung verdeutlichen.

### Beispiel 1

| Wäßrige Zubereitung (Gesichtswasser) | |
|---|---|
| | Gew.-% |
| PEG-40-hydrogenated Castor Oil | 0,811 |
| Dipropylenglycol | 2,534 |
| PEG-8 | 1,521 |
| Na₃ EDTA | 0,253 |
| Polymer JR 125 | 0,025 |
| Vit.-E acetat | 4,0 |
| Kombination E | |
| Vit.-C-palmitat | 1,0 |
| VitaminE -nicotinat | 0,5 |
| Wasser VES | |
| | ad 100,000 |

### Beispiel 2

| Wäßrige Zusammensetzung | |
|---|---|
| | Gew.-% |
| Polyfettsäureester (Cetiol HE) | 16,000 |
| PPG-3-Myristylether (Witconol APM) | 1,000 |
| Propylenglycol | 3,000 |
| Glycerin | 24,000 |
| Alpha - Glycosylrutin | 1,6 |
| L-Cystin | 3,8 |
| Heptadecensäure | 0,1 |
| L-Alanylhistidin | 0,6 |
| Wasser VES | ad 100,000 |

### Beispiel 3

| Hydrogel (Polyacrylatgel) | |
|---|---|
| | Gew.-% |
| Acrylsäurepolymerisat (Carbopol 934) | 1,000 |
| Tris(hydroxymethylamino)methan (Tris) | 1,000 |
| Glycerin | 2,000 |
| Propylenglycol | 2,000 |
| Kombination F | |
| Vit.-E acetat | 3,00 |
| Vit.-A palmitat | 2,60 |
| L-Tryptophan | 2,00 |
| Ölsäure | 0,3 |
| Wasser VES | ad 100,000 |

### Beispiel 4

| Hochwasserhaltige Zubereitung (sehr weich) | |
|---|---|
| | Gew.-% |
| Ceteareth (Cremophor A 25) | 0,100 |
| Cetearyl Alcohol (Lanette O) | 0,400 |
| Vaseline, DAB 9 | 12,500 |
| Mineralöl, DAB 9 | 11,000 |
| Ceteareth-6-stearylalkohol (Cremophor A6) | 6,000 |
| Kombination A: | |
| Superoxyddismutase | 0,008 |
| Gurkenextrakt | 2,90 |
| L - Methionin | 0,70 |
| Wasser VES | ad 100,000 |

### Beispiel 5

| Hochwasserhaltige Zubereitung (weich) | |
|---|---|
| | Gew.-% |
| Ceteareth-25 (Cremophor A25) | 1,500 |
| Cetearyl Alcohol (Lanette O) | 8,500 |
| Kombinationen A + C | |
| Katalase | 0,0005 |
| Glutathionperoxidase | 0,0002 |
| Se - Methionin | 0,8 |
| Wasser VES | ad 100,000 |

### Beispiel 6

| Hochwasserhaltige Zubereitung (weich) | |
|---|---|
| | Gew.-% |
| Ceteareth-25 (Cremophor A25) | 2,000 |
| Cetearylalcohol (Lanette O) | 8,000 |
| Vaseline, DAB 9 | 10,000 |
| Mineralöl, DAB 9 | 10,000 |
| Kombination D: | |
| Panthenol | 1,00 |
| Kaffeesäure | 0,50 |
| Glutathionperoxidase | 0,002 |
| Wasser VES | ad 100,000 |

### Beispiel 7

| Hochwasserhaltige Zubereitung (mittelfest) | |
|---|---|
| | Gew.-% |
| Ceteareth-25 | 3,000 |
| Cetearyl Alcohol (Lanette O) | 17,000 |
| Kombination B | |
| Glutathionreductase p- Isoenzym | 0,0001 |
| Thiaminpyrophosphat | 0,002 |
| NADPH | 0,004 |
| Kaffee-Extrakt | 2,95 |
| Wasser VES | ad 100,000 |

### Beispiel 8

| Dünnflüssige Lotion | |
|---|---|
| | Gew.-% |
| Ceteareth-25 (Cremophor A25) | 1,000 |
| Ceteareth-6-stearylalcohol (Cremophor A6) | 1,000 |
| Glycerin-mono-distearat (Tegin normal) | 2,000 |
| Cetylalcohol | 1,000 |
| Isopropylmyristat | 1,450 |
| Glycerin | 1,000 |
| Polyvinylpyrrolidon | 0,500 |
| Kombination A: | |
| Cholsäure | 0,06 |
| Hamamelisextrakt | 0,09 |
| Wasser VES | ad 100,000 |

### Beispiel 9

| Dickflüssige Lotion | |
|---|---|
| Gew.-% | |
| Ceteareth 25 (Cremophor A25) | 2,000 |
| Cetearyl Alcohol (Lanette O) | 3,000 |
| Mineralöl, DAB 9 | 5,000 |
| Propylenglycol | 3,000 |
| Polyvinylpyrrolidon | 0,500 |
| Kombination A, B und D | A: 14,75 |
| | B: 9,52 |
| | D: 12,50 |
| Harnstoff | 4,25 |
| Wasser VES | ad 100,000 |

### Beispiel 10

| W/O-Crème | |
|---|---|
| | Gew.-% |
| Glycerinsorbitanfettsäureester | |
| (Arlacel 481) | 6,000 |
| Mikrokristallines Wachs (Lunacera M) | 1,000 |
| Neutralöl | 3,000 |
| Paraffinöl | 19,000 |
| Magnesiumstearat | 1,000 |
| Propylenglycol | 3,700 |
| Magnesiumsulfat (MgSO₄*7 H₂O) | 0,700 |
| Kombination A, B und C | |
| Wasser VES | ad 100,000 |

### Beispiel 11

| W/O-Emulsion | |
|---|---|
| | Gew.-% |
| Polyoxyethylen-Glycerin-Sorbitan-Fettsäureester (Arlacel 988) | 3,600 |
| Polyoxyethylen-Fettsäureester | |
| (Arlacel 989) | 1,400 |
| Cetearyl Alcohol (Lanette O) | 2,000 |
| Mineralöl, DAB 9 | 20,000 |
| Paraben-Mischung | nach Belieben |
| Magnesiumsulfat (MgSO₄*7 H₂O) | 0,700 |
| Kombination A + D + F | |
| CaCl₂ | 0,85 |
| Wasser VES | ad 100,000 |

### Beispiel 12

| W/O-Lotion | Gew. % |
|---|---|
| Glycerinsorbitanfettsäureester | |
| (Arlacel 481) | 1,300 |
| Polyoxyethylen-Fettsäureester | |
| (Arlacel 989) | 3,700 |
| Neutralöl (Miglyol) | 6,000 |
| Paraffinöl, DAB 9 | 14,000 |
| Propylenglycol | 3,800 |
| Magnesiumsulfat (MgSO₄*7 H₂O) | 0,700 |
| Liponsäure | 1,50 |
| Kombination A + D | |
| Wasser VES | ad 100,000 |

### Beispiel 13

| O/W-Emulsion | Gew. % |
|---|---|
| Cetearyl Alcohol (Lanette O) | 3,000 |
| Mineralöl, DAB 9 | 25,000 |
| Paraben-Mischung | nach Belieben |
| Kombination C+D | |
| Wasser VES | ad 100,000 |

### Beispiel 14

| O/W-Emulsion | Gew.-% |
|---|---|
| Polysorbate-60 (Tween 60) | 3,000 |
| Sorbitan Stearate (Arlacel 60) | 2,000 |
| Cetearyl Alcohol (Lanette O) | 3,000 |
| Mineralöl, DAB 9 | 25,000 |
| Paraben-Mischung | nach Belieben |
| Ölsäure | 0,30 |
| Kombination A + E | |
| Wasser VES | ad 100,000 |

### Beispiel 15

| Kationenaktive Emulsion | |
|---|---|
| | Gew.-% |
| Distearyldimethylammoniumchlorid | |
| (Genamin DS AC) | 5,000 |
| Vaseline, DAB 9 | 5,000 |
| Isopropylpalmitat | 2,000 |
| Cetylalcohol | 1,000 |
| Siliconöl | 0,100 |
| Propylparaben | 0,100 |
| Methylparaben | 0,100 |
| Glycerin | 4,000 |
| Glucose-6-phosphat | 0,50 |
| Kombination C + D | |
| Wasser VES | ad 100,000 |

### Beispiel 16

| Ionische Emulsion | |
|---|---|
| | Gew.-% |
| Natrium Cetearylsulfat (Emulgade F) | 6,000 |
| Mineralöl, DAB 9 | 25,000 |
| Paraben-Mischung | nach Belieben |
| Glucose-1,6-bisphosphat | 2,00 |
| Kombination D | |
| Wasser VES | ad 100,000 |

### Beispiel 17

| Ionische O/W-Emulsion | |
|---|---|
| | Gew.-% |
| Stearinsäure | 5,000 |
| Cetearyl Alcohol (Lanette O) | 3,000 |
| Mineralöl, DAB 9 | 25,000 |
| Paraben-Mischung | nach Belieben |
| Kombination C | |
| cis-Urocaninsäure | 1,00 |
| Harnstoff | 10,00 |
| Triethanolamin | 1,000 |
| Wasser VES | ad 100,000 |

### Beispiel 18

| Sonnenöl | Gew. % |
|---|---|
| Palmitinsäure | 1,00 |
| 3-(4'-Methylbenzyliden)campher, ("Eusolex 6300", Merck) | 3,00 |
| Myristylalkohol, polyoxypropyliert mit 3 Mol Propylenoxid ("Witconol APM", Witco) | 6,08 |
| C₁₂-C₁₅ -Alkoholbenzoat ("Finsolv TN", Witco) | 15,20 |
| Glycerinmonococoat, polyoxyethyliert mit 7 mol Ethylenoxid ("Cetiol HE" Henkel KGaA | 10,00 |
| Ethanol | 6,50 |
| 2-Octadodecanol | 12,00 |
| Parfüm, Korrigentien, Additive, Stabilisatoren | nach Belieben |
| Kombination A + D | |
| Wasser VES | ad 100,00 |

Die Bestandteile des Sonnenöls werden miteinander vermischt und dabei gegebenenfalls auf 40 bis 50°C zur Homogenisierung erwärmt.

### Beispiel 19

| Sonnengel | Gew. % |
|---|---|
| 2,4,6-Trianilino-(p-carbo-2'-ethylhexyl-1'-oxy)-1,3,5-triazin ("Uvinul" T-150, BASF) | 2,50 |
| Isopropylmyristat | 18,90 |
| C₁₂-C₁₅-Alkoholbenzoat ("Finsolv TN", Witco) | 7,60 |
| Myristylalkohol, polyoxypropyliert mit 3 Mol Propylenoxid ("Witconol APM", Witco) | 30,40 |
| Capryl-/Caprinsäuretriglycerid ("Miglyol-Neutralöl", Dynamit-Nobel) | 19,50 |
| "Bentone-38", Kronos-Titan | 15,00 |
| Propylencarbonat | 2,00 |
| Ethanol | 2,30 |
| Parfüm, Korrigentien, Additive, Stabilisatoren | nach Belieben |
| Kombination C + D + F | |
| Wasser VES | ad 100,0 |

Mit den genannten Bestandteilen wird in üblicher Weise ein Sonnengel hergestellt.

### Beispiel 20

| Hydrogel | Gew. % |
|---|---|
| 2-Phenylbenzimidazol-5-sulfonsäure, ("Eusolex 232", Merck) | 2,70 |
| Allantoin | 2,0g |
| Sorbit fl. ("Karion F", Merck) | 22,0 |
| "Carbopol 934", B.F. Goodrich | 15,0 |
| Tris (hydroxymethyl)aminomechan | 2,70 |
| Propylenglykol | 10,0 |
| Ethanol | 3,0 |
| Kombination B | |
| Parfüm, Korrigentien, Additive, Stabilisatoren | nach Belieben |
| Wasser VES | ad 100,0 |

Mit den genannten Bestandteilen wird in üblicher Weise ein Hydrogel hergestellt.

### Beispiel 21

| | Gew. % |
|---|---|
| Öl-in-Wasser-Emulsion (Sonnencreme) Kombination F | |
| 2-Phenylbenzimidazol-5-sulfonsäure ("Eusolex 232", Merck) | 3,20 |
| Stearylalkohol, der mit 2 Mol Ethylenoxid oxyethyliert ist ("Brij 72", ICI) | 3,00 |
| Stearylalkohol, der mit 21 Mol Ethylenoxid oxyethyliert ist ("Brij 721", ICI) | 2,00 |
| Cetylstearylalkohol | 12,50 |
| Myristylalkohol, polyoxypropyliert mit 3 Mol Propylenoxid ("Witconol APM", Witco) | 6,40 |
| C₁₂-C₁₅-Alkoholbenzoat ("Finsolv TN", Witco) | 11,60 |
| Propylenglykol | 8,50 |
| Tris(hydroxymethyl)aminomethan | 1,40 |
| Parfüm, Korrigentien, Additive, Stabilisatoren | nach Belieben |
| Wasser VES | ad 100,0 |

Die Fettkörper werden auf 80 bis 85°C erwärmt. Die wasserlöslichen Bestandteile werden bei 40°C in Wasser gelöst, beide Phasen unter kräftigem Rühren miteinander vermischt, und unter mäßigerem Rühren läßt man abkühlen.

### Beispiel 22

| Öl-in-Wasser-Emulsion (Sonnencreme) | Gew. % |
|---|---|
| Kombination A + B + D | |
| 2,4,6-Trianilino-(p-carbo-2'-ethylhexyl-1'-oxy)-1,3,5-triazin ("Uvinul T-150", BASF) | 1,80 |
| C₁₂-C₁₅-Alkoholbenzoat ("Finsolv TN", Witco) | 4,70 |
| Cetylstearylalkohol | 3,0 |
| Gemisch aus Stearinsäuremono- und diester des Glycerins, sowie Stearinsäureester von Polyethylenoxid ("Arlacel 165", ICI) | 5,0 |
| Myristylalkohol, polyoxypropyliert mit 3 Mol Propylenoxid ("Witconol APM", Witco) | 18,5 |
| Parfüm, Korrigentien, Additive, Stabilisatoren | nach Belieben |
| Wasser VES | ad 100,0 |

Die Emulsion wird entsprechend vorstehendem Beispiel zubereitet.

### Beispiel 23

| Wasser-in-Öl-Emulsion (Sonnenschutzmilch) | Gew. % |
|---|---|
| Kombination C + F | |
| 1-(4'-tert.Butylphenyl)-3-(4'-methoxyphenyl)propan-1,3-dion ("Parsol 1789", Givaudan) | 1,50 |
| 4-Methoxyzimtsäure-2'-ethylhexylester, ("Parsol MCX", Givaudan) | 3,50 |
| Ester gesättigter Fettsäuren mit Polyethylenoxid ("Arlacel 989", ICI) | 3,70 |

### Beispiel 24

| Wasser-in-Öl-Emulsion (Sonnenschutzmilch) | Gew.% |
|---|---|
| Kombination A+C 4-Methoxyzimtsäure-2'-ethylhexylester, | |
| ("Parsol MCX", Givaudan) 3-(4'-Methylbenzyliden)campher ("Eusolex 6300", Merck) | 1,50 3,0 |
| Ester ungesättigter Fettsäuren mit Glycerin und ("Arlacel 481", ICI) | 6,00 |
| Mikrowachs ("Lunacera 11", Fuller) | 1,00 |
| Capryl-/Caprinsäuretriglycerid ("Miglyol-Neutralöl", Dynamit-Nobel) | 2,00 |
| Myristylalkohol, polyoxypropyliert mit 3 Mol Propylenoxid ("Witconol APM", Witco) | 1,45 |
| C₁₂-C₁₅-Alkoholbenzoat ("Finsolv TN", Witco) | 3,70 |
| Magnesiumstearat | 1,00 |
| Propylenglykol | 3,70 |
| Magnesium-Heptahydrat | 0,70 |
| Parfüm, Korrigentien, Additive, Stabilisatoren | nach Belieben |
| Wasser VES | ad 100,0 |

Die Emulsion wird in der entsprechenden Weise hergestellt wie unter Beispiel 21 beschrieben.

| | |
|---|---|
| Ester ungesättigter Fettsäuren mit Glycerin und Sorbitan ("Arlacel 481", ICI) | 1,30 |
| Myristylalkohol, polyoxypropoxyliert mit 3 Mol Propylenoxid ("Witconol APM", Witco) | 16,00 |
| C₁₂-C₁₅-Alkoholbenzoat ("Finsolv TN", Witco) | 4,00 |
| Magnesiumsulfat-Heptahydrat | 0,70 |
| Parfüm, Korrigentien, Additive, Stabilisatoren | nach Belieben |
| Wasser VES | ad 100,0 |

Die Emulsion wird in der entsprechenden Weise hergestellt wie unter Beispiel 21 beschrieben.

### Beispiel 25

| Wasser-in-Öl-Emulsion (Sonnenschutzmilch) | Gew. % |
|---|---|
| FADH₂ | 0,09 |
| Glucose-1,6-phosphat | 1,23 |
| D-Biotin | 0,04 |
| D-Carnosin | 1,0 |
| Vit.-C-dipalmitat | 2,0 |
| Vit.-E-acetat | 3,0 |
| Phytinsäure | 1,70 |
| Urocaninsäure | 1,30 |
| L-Cystein | 1,57 |
| Dithiopropylgallat | 4,00 |
| 4-Methoxyzimtsäure-2'-ethylhexylester ("Parsol MCX", Givaudan) | 1,50 |
| 3-(4'-Methylbenzyliden)campher ("Eusolex 6300", Merck) | 3,0 |
| Ester ungesättigter Fettsäuren mit Glycerin und Sorbitan ("Arlacel 481", ICI) | 6,00 |
| Mikrowachs ("Lunacera 11", Fuller) | 1,00 |
| Capryl-/Caprinsäuretriglycerid ("Miglyol-Neutralöl", Dynamit-Nobel) | 2,0 |
| Myristylalkohol, polyoxypropyliert mit 3 mol Propylenoxid ("Witconol APM", Witco) | 119,0 |
| C₁₂-C₁₅-Alkoholbenzoat ("Finsolv TN", Witco) | 30,0 |
| Magnesiumstearat | 1,00 |
| Propylenglykol | 3,70 |
| Magnesiumsulfat-Heptahydrat | 7,0 |
| Parfüm, Korrigentien, Additive, Stabilisatoren | nach Belieben |
| Wasser VES | ad 100,0 |

Die Emulsion wird in der gleichen Weise hergestellt wie unter Beispiel 21 beschrieben.

### Beispiel 26

| Kationische Emulsion zur Spülung der Haare | Gew. % |
|---|---|
| Vitamin E -acetat | 16,5 |
| trans - Urocaninsäure | 7,0 |
| Dimethyldistearylammoniumchlorid ("Arosorf TA 100", Rewo) | 5,00 |
| Vaseline | 5,00 |
| Isopropylpalmitat | 2,0 |
| Cetylalkohol | 10,0 |
| Wasser | 8,33 |
| Glycerin | 4,0 |
| Parfüm, Korrigentien, Additive, Stabilisatoren | nach Belieben |
| Wasser | ad 100,0 |

Mit den angegebenen Bestandteilen wird in üblicher Weise eine Haarspülung hergestellt.

Zur Herstellung der topischen Zubereitungen werden die Wirkstoffe und Wirkstoffkombinationen in der wäßrigen Phase oder in der Fettphase gelöst und in bekannter Weise weiterverarbeitet.

### Herstellung von Kapseln

Kapseln, welche die nachstehend angegebenen Bestandteile enthalten, werden nach bekannten Arbeitsweisen hergetellt. Diese sind für die Behandlung der vorstehend genannten Krankheiten in Dosierungsmengen von jeweils einer Kapsel einmal oder mehrmals täglich geeignet.

### Beispiel 27

| Bestandteile | Gew. % |
|---|---|
| Vit. E -palmitat | 5,0 |
| Ca-Ascorbylphosphat | 5,0 |
| DL-Carnosin | 2,5 |
| Kombination A | |
| Kombination D | |

## Patentansprüche

1. Verwendung von
a) einer Verbindung oder mehrerer Verbindungen aus der Gruppe der Flavonoide oder
b) einer Wirkstoffkombination, enthaltend eine Verbindung oder mehrere Verbindungen, ausgewählt aus der Gruppe der Flavonoide in Kombination mit einer Verbindung oder mehreren Verbindungen, ausgewählt aus der Gruppe der Zimtsäurederivate, und gegebenenfalls zusätzlich jeweils einer Verbindung oder mehreren Verbindungen aus einer der Gruppen oder mehreren der Gruppen
c) der Antioxidantien oder
d) der endogenen Energiestoffwechselmetaboliten oder
e) der endogenen enzymatischen Antioxydansysteme und deren synthetischen Derivaten (Mimics) oder
f) der antimikrobiellen Wirksysteme oder
g) der antiviralen Wirksysteme oder
h) Wirkstoffen der bekannten, konventionellen Behandlungsformen
jeweils zur Herstellung eines dermatologischen und/oder pharmazeutischen Mittels zur Behandlung oder prophylaktischen Behandlung von hyperreaktiver und für Dermatiden prädisponierter Haut oder defizitärer, hypoaktiver Haut oder Dermatosen.

2. Verwendung gemäß Anspruch 1, **dadurch gekennzeichnet, daß** die Flavonoide gewählt sind aus der Gruppe Quercitin, Rutin, Chrysin, Kaempferol, Myricetin, Rhamnetin, Apigenin, Luteolin, Naringin, Hesperidin, Naringenin, Hesperitin, Morin, Phloridzin, Diosmin, Fisetin, Vitexin, Neohesperidin Dihydrochalkon, Flavon, Glucosylrutin und Genistein, alpha-Glucosylrutin, alpha-Glucosylmyrictrin, alpha-Glucosylisoquercitrinitrin und alpha-Glucosylquercitrin, alpha-Glucosylrutin, alpha-Glucosylmyrictrin, alpha-Glucosylisoquercitrinitrin und alpha-Glucosylquercitrin.

3. Verwendung gemäß Anspruch 1, **dadurch gekennzeichnet, daß** die Kombination b) verwendet wird.

4. Verwendung gemäß Anspruch 1, **dadurch gekennzeichnet, daß** Hydroxyzimtsäure verwendet werden.

5. Verwendung gemäß Anspruch 1, **dadurch gekennzeichnet, daß** Zimtsäurederivate der allgemeinen Formel 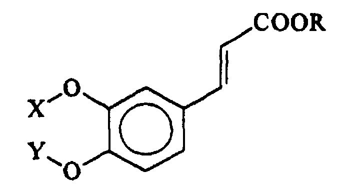 und/oder wirksame Mengen an Zimtsäurederivaten der allgemeinen Formel 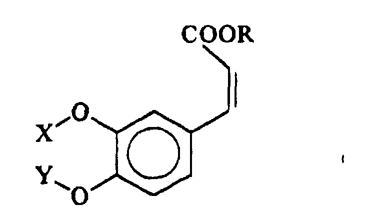 verwendet werden, wobei die Gruppen X, Y und R unabhängig voneinander gewählt werden können aus der Gruppe H, verzweigtes bzw. unverzweigtes Alkyl mit 1 - 18 C-Atomen.

6. Verwendung gemäß Anspruch 1, **dadurch gekennzeichnet, daß** Kaffesäure und/oder Ferulasäure verwendet werden.

7. Verwendung gemäß Anspruch 1, **dadurch gekennzeichnet, daß** Kombinationen b) mit alpha-Glucosylrutin und/oder Ferulasäure verwendet werden.

## Claims

1. Use of
a) a compound or several compounds from the group consisting of flavonoids, or
b) an active compound combination comprising a compound or several compounds chosen from the group consisting of flavonoids, in combination with a compound or several compounds chosen from the group consisting of cinnamic acid derivatives, and if appropriate additionally in each case a compound or several compounds from one of the groups or several of the groups
c) of the antioxidants or
d) of the endogenous energy metabolism metabolites or
e) of the endogenous enzymatic antioxidant systems and synthetic derivatives thereof (mimics) or
f) of the antimicrobial action systems or
g) of the antiviral action systems or
h) active compounds of the known, conventional treatment forms
in each case for the preparation of a dermatological and/or pharmaceutical agent for the treatment or prophylactic treatment of hyperreactive skin predisposed to dermatitis or deficient, hypoactive skin or dermatoses.

2. Use according to Claim 1, **characterized in that** the flavonoids are chosen from the group consisting of quercitin, rutin, chrysin, kaempferol, myricetin, rhamnetin, apigenin, luteolin, naringin, hesperidin, naringenin, hesperitin, morin, phloridzin, diosmin, fisetin, vitexin, neohesperidin dihydrochalcone, flavone, glucosylrutin and genistein, alpha-glucosylrutin, alpha-glucosylmyricbrin, alpha-glucosylisoquercitrinitrin and alpha-glucosylquercitrin, alpha-glucosylrutin, alpha-glucosylmyrictrin, alpha-glucosylisoquercitrinitrin and alpha-glucosylquercitrin.

3. Use according to Claim 1, **characterized in that** combination b) is used.

4. Use according to Claim 1, **characterized in that** hydroxycinnamic acid is used.

5. Use according to Claim 1, **characterized in that** cinnamic acid derivatives of the general formula 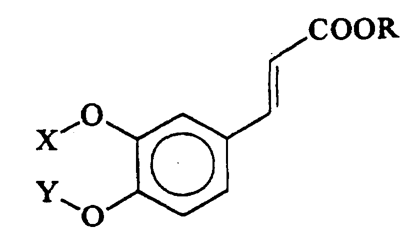 and/or active amounts of cinnamic acid derivatives of the general formula 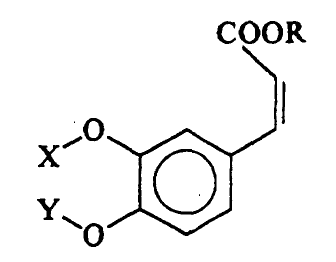 wherein the groups X, Y and R independently of one another can be chosen from the group consisting of H and branched or unbranched alkyl having 1 - 18 C atoms, are used.

6. Use according to Claim 1, **characterized in that** caffeic acid and/or ferulic acid are used.

7. Use according to Claim 1, **characterized in that** combinations b) with alpha-glucosylrutin and/or ferulic acid are used.

## Revendications

1. Utilisation de
a) un composé ou plusieurs composés parmi le groupe constitué de flavonoïdes ou
b) une combinaison d'ingrédients actifs comprenant un composé ou plusieurs composés choisis parmi le groupe constitué de flavonoïdes, en combinaison avec un composé ou plusieurs composés choisis parmi le groupe constitué de dérivés de l'acide cinnamique, et éventuellement en outre, dans chaque cas, un composé ou plusieurs composés parmi l'un des groupes ou plusieurs des groupes
c) les antioxydants ou
d) les métabolites endogènes du métabolisme énergétique ou
e) les systèmes antioxydants enzymatiques endogènes et leurs dérivés synthétiques (agents mimétiques) ou
f) les systèmes d'action antimicrobiens ou
g) les systèmes d'action antiviraux ou
h) des ingrédients actifs des formes de traitement traditionnelles connues
dans chaque cas pour la préparation d'une composition dermatologique et/ou pharmaceutique destinée au traitement ou au traitement prophylactique d'une peau hyper réactive et prédisposée à une dermatite ou d'une peau hypo-active déficiente ou de dermatoses.

2. Utilisation selon la revendication 1, **caractérisée en ce que** les flavonoïdes sont choisis parmi le groupe constitué de la quercitine, de la rutine, de la chrysine, du kaempférol, de la myricétine, de la rhamnétine, de l'apigénine, de la lutéoline, de la naringine, de l'hespéridine, de la naringénine, de l'hespéritine, de la morine, de la phloridzine, de la diosmine, de la fisétine, de la vitexine, de la néohespéridine-dihydrochalcone, de la flavone, de la glucosylrutine et de la génistéine, de l'alpha-glucosylrutine, de l'alpha-glucosylmyrictrine, de l'alpha-glucosylisoquercitrinitrine et de l'alpha-glucosylquercitrine, de l'alpha-glucosylrutine, de l'alpha-glucosylmyrictrine, de l'alpha-glucosylisoquercitrinitrine et de l'alpha-glucosylquercitrine.

3. Utilisation selon la revendication 1, **caractérisée en ce que** la combinaison b) est utilisée.

4. Utilisation selon la revendication 1, **caractérisée en ce que** l'acide hydroxycinnamique est utilisé.

5. Utilisation selon la revendication 1, **caractérisée en ce que** des dérivés de l'acide cinnamique de formule générale 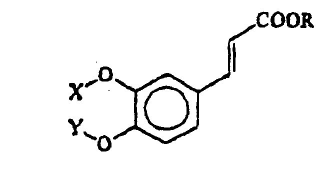 et/ou des quantités actives de dérivés de l'acide cinnamique de formule générale 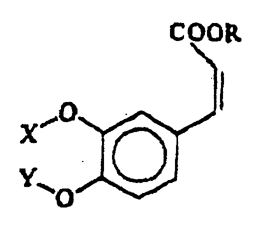 sont utilisés, les groupes X, Y et R pouvant être choisis, indépendamment les uns des autres parmi le groupe constitué de H, d'un alkyle ramifié et d'un alkyle non ramifié ayant de 1 à 18 atomes de carbone.

6. Utilisation selon la revendication 1, **caractérisée en ce que** l'acide caféique et/ou l'acide férulique sont utilisés.

7. Utilisation selon la revendication 1, **caractérisée en ce que** des combinaisons b) avec l'alpha-glucosylrutine et/ou l'acide férulique sont utilisées.
